# EUROPEAN PATENT SPECIFICATION

(11) **EP 4 518 796 B1**
(45) Date of publication and mention of the grant of the patent: **09.12.2026**
(21) Application number: 23799914.9
(22) Date of filing: 02.05.2023
(51) Int. Cl.: A61F 2/01

(54) **EMBOLIC FILTER CATHETER**
EMBOLIEFILTERKATHETER
CATHÉTER À FILTRE EMBOLIQUE

(30) Priority: 02.05.2022 US 202263337340 P
(43) Date of publication of application: 12.03.2025
(73) Proprietor: EmStop Inc., Brainerd, MN 56401 (US)
(72) Inventor: BUSTER, Brenden, Brainderd, Minnesota 56401 (US); WISGERHOF, Taylor, New Hope, Minnesota 55427 (US); SLETTEHAUGH, Zoey, Minneapolis, Minnesota 55427 (US); ADAIR, Jeremy, Minneapolis, Minnesota 55413 (US); GUSTAFSON, Alex, Minneapolis, Minnesota 55413 (US); FEDDEMA, David, Maple Grove, Minnesota 55369 (US)
(74) Representative: Mathys & Squire
(86) International application number: PCT/US2023/020674
(87) International publication number: WO 2023/215273

(56) References cited:
- US-A1- 2013 253 570
- US-A1- 2017 165 062
- US-A1- 2017 325 955
- US-A1- 2020 107 923
- US-A1- 2021 045 759

## Description

This application claims the benefit of U.S. Provisional Patent Application No. 63/337,340 filed May 2, 2022.

### BACKGROUND

Transcatheter aortic valve replacement (TAVR) is a proven strategy for the treatment of severe aortic stenosis that has been validated for use in patients who are not eligible for surgical aortic valve replacement (SAVR) due to patient frailty or associated high operative risk. TAVR with the use of a self-expanding or balloon-expanded bioprosthetic valve has been FDA-approved for commercial use in the US in selected patients. TAVR is rapidly becoming the method of choice to treat aortic stenosis in patients deemed to be at increased risk of death if offered a traditional surgical aortic valve replacement. Patients presently selected for TAVR, however, are most often elderly with frailty and a number of comorbidities. The femoral artery is generally the first choice for access to the aortic valve. In patients with significant arterial occlusive disease, however, marked tortuosity of the ileo femoral system and/or significant at risk atheromatous plaque within the native aorta and/or aneurysmal disease may present significant risk for femoral access such that alternate access TAVR is preferable. An alternative route has been proposed several years ago in the form of a trans-apical (TA) approach through the apex of the left ventricle exposed through a left lateral thoracotomy. The TA approach, however, requires opening the left chest in patients having potential pulmonary dysfunction and the rate of bleeding complications may be higher than that observed after traditional trans-femoral (TF) approach. In the search for yet another alternative to compromised peripheral arterial vascular access, a direct trans-aortic (TAo) route has been described in a limited number of cases since 2010. In a recent report, the cases performed through a TAo route represented only 4% of the TAVR cases performed by 2013.

Although results have been encouraging with TAVR, the risk of stroke has been demonstrated to be significantly higher with TAVR relative to SAVR. Clinically observed stroke (CVA) underestimates the prevalence of embolic events inherent with TAVR. During TAVR, stent and implanted valve expansion (with or without the use of a balloon) results in native valve compression and radial leaflet displacement that leads to the liberation of tissue and particulate matter that travels distally in the arterial tree. Some of the debris lodges in terminal branches of cerebral vessels and will be evidenced with new onset stroke. Other debris released at the time of TAVR lodge in vessels of the peripheral circulation, renal circulation, coronary circulation, and mesenteric circulation. These patients may manifest clinical scenario of renal failure, mesenteric ischemia, peripheral ischemia, and/or myocardial infarction. Other patients may not have acute clinical deterioration but may suffer late effects due to impaired functional reserve related to sub-clinical embolic events. The occurrence of embolic events during TAVR is a significant impediment to offering the technique to larger lower risk groups of patients.

A number of different approaches have been developed for embolic protection. Existing embolic protection devices are primarily configured for deflecting embolic material from the brachiocephalic vessels or capturing embolic material within the brachiocephalic vessels. There are a number of difficulties with these existing embolic protection devices. First, deployment of the devices requires additional time and can conflict with the performance of the valve implantation procedure. Second, deployment of the devices may lead to additional vessel trauma and liberation of embolic material. Third, the deployment of the devices may be difficult and stability of deployment may make protection less than reliable. Fourth, the devices may not protect the brain from all sources of blood flow and particularly posterior cerebral blood flow is not filtered. Fifth, systemic embolization may still occur that may lead to intestinal, renal, and/or peripheral manifestations of ischemic gut, renal insufficiency and/or peripheral ischemia. Sixth, coronary embolization and myocardial infarction may occur due to proximal embolization.

US 2020/107923 A1 discloses a method for deploying embolic material capture element in blood vessel, involves distally advancing deployment cap assembly relative to dilator sheath to release distal end portion of embolic material capture element from engagement with dilator assembly.

### BRIEF SUMMARY

The following presents a simplified summary of some embodiments of the invention in order to provide a basic understanding of the invention. This summary is not an extensive overview of the invention. It is not intended to identify key/critical elements of the invention or to delineate the scope of the invention. Its sole purpose is to present some embodiments of the invention in a simplified form as a prelude to the more detailed description that is presented later.

In many embodiments, an embolic material filter catheter includes an embolic material filter assembly that is deployable within a blood vessel downstream of a treatment site to capture embolic material released from the treatment site. In many embodiments, the deployment of the embolic material filter assembly is accomplished via a deployment sequence in which a distal end portion of the embolic material filter assembly is held in a collapsed configuration while a proximal end portion of the embolic material filter assembly is advanced distally toward the distal end portion of the embolic material filter assembly, thereby expanding a middle portion of the embolic material filter assembly. In many embodiments, following expansion of the middle portion of the embolic material filter assembly, the distal end portion of the embolic material filter assembly is released and self-expands into engagement with the blood vessel. In some embodiments, the embolic material filter assembly is constrained in the insertion configuration via axial tension applied to the embolic material filter assembly, thereby enabling the embolic material filter catheter to have a reduced diameter and/or increased flexibility since a retaining sheath is not required to retain the embolic material filter assembly in the insertion configuration. The embolic material filter catheter can be configured for use in any suitable procedure. For example, the embolic material filter catheter can be used during implantation of a prosthetic aortic valve in which the embolic material filter assembly is deployed in a patient's aorta downstream of the patient's aortic valve to capture embolic material released during implantation of the prosthetic aortic valve. In many embodiments, the embolic material filter catheter includes a lumen into which a delivery catheter for the prosthetic valve can be inserted to advance the prosthetic valve to an implantation site upstream of the deployed embolic material filter assembly. In many embodiments, the lumen is configured to accommodate extraction of embolic material captured by the embolic material filter assembly. In some embodiments, such as embodiments sized for insertion through the femoral artery, removal of embolic material through the lumen of the embolic material filter catheter may not be possible while the delivery catheter for the prosthetic valve is accommodated in the lumen. In such embodiments, removal of embolic material through the lumen of the embolic material filter catheter can be accomplished following removal of the delivery catheter for the prosthetic valve from the lumen of the embolic material filter catheter. The embolic material filter catheter and related treatment catheters, devices, and methods are especially suited for use in TAVR via any suitable access (including, but not limited to, femoral, direct aortic access, brachiocephalic, subclavian, axillary or carotid arteries) that enables accurate positioning of the prosthetic aortic valve.

Thus, in one aspect, an embolic material filter catheter includes an introducer sheath, an inner sheath, an embolic material filter assembly, and a dilator assembly. The introducer sheath defines an introducer sheath lumen. The inner sheath is slidably disposed in the introducer sheath lumen and defines an inner sheath lumen. The embolic material filter assembly has a proximal end portion and a distal end portion. The proximal end portion is attached to a distal end portion of the inner sheath. The embolic material filter assembly is reconfigurable between an insertion configuration and a deployed configuration. The embolic material filter assembly is configured to interface with an inner surface of a blood vessel in the deployed configuration. The embolic material filter assembly is configured to filter embolic material from blood flowing through the embolic material filter assembly. The dilator assembly has a holding configuration and a non-holding configuration. The dilator assembly includes a dilator shaft, a dilator distal end member, and dilator pull wires. The dilator shaft is configured to be extended through the inner sheath lumen. The dilator distal end member is attached to a distal end of the dilator shaft. The distal end portion of the embolic material filter assembly is restrained in the insertion configuration via engagement with the dilator pull wires and the dilator distal end member in the holding configuration. The dilator pull wires are configured to be translated proximally relative to the dilator distal end member to reconfigure the dilator assembly from the holding configuration to the non-holding configuration to release the distal end portion of the embolic material filter assembly from engagement with the dilator pull wires and the dilator distal end member to reconfigure the embolic material filter assembly to the deployed configuration. The dilator assembly is removable from the inner sheath lumen while the embolic material filter assembly is in the deployed configuration via proximal retraction of the dilator assembly relative to the inner sheath.

In many embodiments, dilator assembly is configured to inhibit inducing trauma to the patient's vasculature. For example, the dilator distal end member can include dilator pull wire recesses. Each of the dilator pull wires can include a respective dilator pull wire distal end portion that is disposed within a corresponding one of the dilator pull wire recesses when the dilator assembly is in the holding configuration. Each of the dilator pull wire distal end portions is pulled out of the corresponding one of the dilator pull wire recesses during reconfiguration of the dilator assembly from the holding configuration to the non-holding configuration.

In many embodiments, the embolic material filter catheter is operable to capture the embolic material filter assembly within the introducer sheath prior to removal of the embolic material filter assembly from the patient. For example, the embolic material filter assembly can be reconfigurable from the deployed configuration to a captured configuration in which the embolic material filter assembly is disposed in the introducer sheath lumen via proximal retraction of the inner sheath relative to the introducer sheath.

In many embodiments, the embolic material filter assembly can be maintained in the insertion configuration when disposed distal to the introducer sheath. The embolic material filter assembly can be restrained to conform to an outer surface of the dilator shaft from the proximal end portion of the embolic material filter assembly to the distal end portion of the embolic material filter assembly when the embolic material filter assembly is in the insertion configuration. The embolic material filter assembly can have an outer surface that extends between the proximal end portion of the embolic material filter assembly and the distal end portion of the embolic material filter assembly. The outer surface of the embolic material filter assembly is disposable distal to the introducer sheath with the embolic material filter assembly in the insertion configuration. The embolic material filter assembly can be configured to be retained in the insertion configuration at least partially via axial tension imparted into the embolic material filter assembly via the dilator assembly and the inner sheath.

In many embodiments, the inner sheath is sized to accommodate a treatment catheter. For example, the inner sheath can be sized to accommodate insertion of a treatment catheter into the inner sheath lumen and advancement of a distal portion of the treatment catheter to a position distal to the distal end portion of the embolic material filter assembly in the deployed configuration. The distal end portion of the treatment catheter can be configured for accomplishing a surgical task.

In some embodiments, the embolic material filter assembly is configured for use in a patient's aorta. For example, the embolic material filter assembly can be configured to, in the deployed configuration, interface with a patient's aorta and substantially block flow of embolic material through the patient's aorta past the embolic material filter assembly. The treatment catheter can be configured for deploying a prosthetic aortic valve.

In some embodiments, the embolic material filter assembly includes an outer scaffold and an inner filter mounted to the outer scaffold. The inner filter can be configured to filter embolic material from blood flowing through the inner filter.

In some embodiments, the embolic material filter catheter is configured to be coupled with an embolic material extraction device. The embolic material extraction device can be operable to draw embolic material through the inner sheath lumen while the embolic material filter assembly is in the deployed configuration.

In some embodiments, the embolic material filter assembly includes an outer scaffold and an inner filter attached to the outer scaffold. The outer scaffold can be configured to radially expand into contact with a blood vessel along which embolic material is blocked from traversing. The inner filter can be configured to prevent emboli of greater than a particular size from passing through the inner filter. The outer scaffold can include distally extending loops of wires configured for atraumatic engagement of the blood vessel.

In some embodiments, a middle portion of the embolic material filter assembly has a middle portion external diameter in the deployed configuration. The distal end portion of the embolic material filter assembly has a distal end portion external diameter in the deployed configuration. In some embodiments, the middle portion external diameter is less than the distal end portion external diameter.

In many embodiments, the embolic material filter assembly includes an outer scaffold and an inner filter. The inner filter can be separated from the outer scaffold by an intervening annular space in the deployed configuration along a length of the inner filter.

In some embodiments, a proximal end portion of the outer scaffold is formed by proximally extending unbraided outer scaffold wire segments that are bonded to an outer surface of the inner sheath. Each of the unbraided outer scaffold wire segments can have a reduced diameter produced via electro-polishing.

In another aspect, a method of deploying an embolic material filter assembly in a blood vessel includes constraining a proximal end portion of an embolic material filter assembly via attachment to a distal end portion of an inner sheath having an inner sheath lumen. A distal end portion of the embolic material filter assembly is restrained in an insertion configuration of the embolic material filter assembly via restraint of the distal end portion of the embolic material filter assembly with a dilator assembly that extends through the inner sheath lumen. The dilator assembly has a holding configuration and a non-holding configuration. The dilator assembly includes a dilator shaft, a dilator distal end member, and dilator pull wires. The embolic material filter assembly is advance in the insertion configuration through the blood vessel. The dilator pull wires are retracted proximally relative to the dilator distal end member to reconfigure the dilator assembly from the holding configuration to the non-holding configuration to release the distal end portion of the embolic material filter assembly from engagement with the dilator pull wires and the dilator distal end member to reconfigure the embolic material filter assembly to a deployed configuration via self-expansion of the embolic material filter assembly.

In many embodiments, the method includes capturing the embolic material filter assembly prior to removal of the embolic material filter assembly from the patient. For example, the method can include capturing the embolic material filter assembly via proximal retraction of the inner sheath relative to an introducer sheath to retract the embolic material filter assembly into an introducer sheath lumen of the introducer sheath.

In many embodiments of the method, the embolic material filter assembly has an outer surface that extends between the proximal end portion of the embolic material filter assembly and the distal end portion of the embolic material filter assembly. The outer surface of the embolic material filter assembly can be disposable distal to the introducer sheath when the embolic material filter assembly is advanced through the blood vessel in the insertion configuration. The embolic material filter assembly can conform to an outer surface of the dilator assembly from the proximal end portion of the embolic material filter assembly to the distal end portion of the embolic material filter assembly when the embolic material filter assembly is in the insertion configuration. The embolic material filter assembly can be retained in the insertion configuration at least partially via axial tension imparted into the embolic material filter assembly via the dilator assembly and the inner sheath.

In many embodiments, the method includes advancing a distal portion of a treatment catheter through the inner sheath lumen to a position distal to the proximal end portion of the embolic material filter assembly in the deployed configuration. A surgical task can be accomplished distal to the proximal end portion of the embolic material filter assembly in the deployed configuration via the treatment catheter.

In many embodiments, the method includes interfacing the embolic material filter assembly in the deployed configuration with a patient's aorta. The method can further include blocking flow of embolic material through the patient's aorta past the embolic material filter assembly. The method can further include deploying a prosthetic aortic valve via the treatment catheter.

In many embodiments, the method includes reconfiguring the embolic material filter assembly from the insertion configuration to an intermediate deployment configuration. For example, the embolic material filter assembly can be reconfigured from the insertion configuration to the intermediate deployment configuration by expanding a middle portion of the embolic material filter assembly disposed between the proximal end portion of the embolic material filter assembly and the distal end portion of the embolic material filter assembly via distal advancement of the inner sheath toward the distal end portion of the embolic material filter assembly constrained by the dilator assembly.

In many embodiments, the method includes drawing embolic material through the inner sheath lumen while the embolic material filter assembly is in the deployed configuration. The embolic material can be drawn through the inner sheath lumen by an embolic material extraction device fluidly coupled with the inner sheath lumen.

In many embodiments of the method, the embolic material filter assembly includes an outer scaffold and an inner filter attached to the outer scaffold. The outer scaffold can be configured to radially expand into contact with a blood vessel along which embolic material is blocked from traversing by the inner filter. The inner filter can be configured to prevent emboli of greater than a particular size from passing through the inner filter. The outer scaffold can include distally extending loops of wires configured for atraumatic engagement of the blood vessel.

In many embodiments of the method, the embolic material filter assembly includes suture loops. Each of the suture loops can pass through each of the distal extending loops of wires in a respective set of the distal extending loops of wires and be engaged with a respective one of the dilator pull wires in the insertion configuration.

In many embodiments of the method, a middle portion of the embolic material filter assembly has a middle portion external diameter in the deployed configuration. The distal end portion of the embolic material filter assembly can have a distal end portion external diameter in the deployed configuration and the middle portion external diameter can be less than the distal end portion external diameter.

In many embodiments of the method, the embolic material filter assembly includes an outer scaffold and an inner filter. The inner filter can be separated from the outer scaffold by an intervening annular space in the deployed configuration along a length of the inner filter.

In many embodiments of the method, the embolic material filter assembly includes an outer scaffold and an inner filter. A proximal end portion of the outer scaffold can be formed by proximally extending unbraided wire segments of the outer scaffold that are bonded to an outer surface of the inner sheath. Each of the proximally extending unbraided wire segments of the outer scaffold can have a reduced diameter produced via electro-polishing.

In another aspect, an embolic material filter catheter includes an introducer sheath, an inner sheath, an embolic material filter assembly, and a dilator assembly. The introducer sheath defines an introducer sheath lumen. The inner sheath is slidably disposed in the introducer sheath lumen and defines an inner sheath lumen. The embolic material filter assembly is coupled to a distal portion of the inner sheath. The embolic material filter assembly includes an outer scaffold and an inner filter. The embolic material filter assembly is reconfigurable from an insertion configuration to a deployed configuration. The insertion configuration accommodates insertion of the distal portion of the inner sheath and the embolic material filter assembly into a blood vessel of a patient to position the embolic material filter assembly downstream of a treatment site. In the deployed configuration, the outer scaffold has an outer circumference configured to interface with the blood vessel and positions the inner filter to filter embolic material from blood flowing through the embolic material filter assembly. The outer scaffold undergoes a reduction in length during a reconfiguration of the embolic material filter assembly from the insertion configuration to the deployed configuration. The inner filter includes body pleats in the deployed configuration that are configured to accommodate the reduction in length of the outer scaffold during the reconfiguration of the embolic material filter assembly from the insertion configuration to the deployed configuration. The dilator assembly has a holding configuration and a non-holding configuration. In the holding configuration, the dilator assembly restrains a distal end portion of the embolic material filter assembly in the insertion configuration. Reconfiguration of the dilator assembly from the holding configuration to the non-holding configuration releases the distal end portion of the embolic material filter assembly to accommodate reconfiguration of the embolic material filter assembly to the deployed configuration.

In the insertion configuration, the outer scaffold can extend distally from the distal portion of the inner sheath by at least 1 inch. In some embodiments, the reduction in length of the outer scaffold during the reconfiguration of the embolic material filter assembly from the insertion configuration to the deployed configuration is at least 0.5 inch.

The body pleats can have any suitable configuration. For example, in some embodiments, the body pleats are arranged axially-symmetric to a centerline of the embolic material filter assembly. In other embodiments, the body pleats extend helically around a centerline of the embolic material filter assembly. In some embodiments, the inner filter does not comprise the body pleats in the insertion configuration.

In some embodiments, a distal end of the inner filter has distal end segments arranged in a zig-zag manner in the deployed configuration. In some embodiments, the distal end segments are aligned with and woven into a braid of the outer scaffold to secure the inner filter to the outer scaffold to inhibit passage of emboli around the inner filter and to minimize a resulting combined thickness of the embolic material filter assembly at the distal end of the inner filter.

In many embodiments, the embolic material filter assembly is configured to be retained in the insertion configuration at least partially via axial tension imparted into the embolic material filter assembly via the dilator assembly and the inner sheath. In many embodiments, the inner filter is formed to have the body pleats when not subjected to axial tension imparted into the embolic material filter assembly via the dilator assembly. In some embodiments, the body pleats are formed in the inner filter via heat setting.

In many embodiments, the embolic material filter assembly can be captured via the introducer sheath prior to removal of the embolic material filter assembly from the patient. For example, the embolic material filter assembly can be reconfigurable from the deployed configuration to the captured configuration via proximal retraction of the inner sheath relative to the introducer sheath.

In many embodiments, the embolic material filter assembly has configurational aspects directed to reducing the diametrical size of the embolic material filter assembly during insertion into the patient's vasculature. For example, in many embodiments, the embolic material filter assembly is disposable distal to the introducer sheath with the embolic material filter assembly in the insertion configuration. In many embodiments, the embolic material filter assembly is conformable to an outer surface of the dilator assembly when the embolic material filter assembly is in the insertion configuration and disposed distal to the introducer sheath.

In many embodiments, the inner sheath accommodates insertion of a treatment catheter into the inner sheath lumen and advancement of a distal portion of the treatment catheter to a position distal to the distal end portion of the embolic material filter assembly in the deployed configuration. The treatment catheter can have any suitable configuration. For example, the distal end portion of the treatment catheter can be configured for accomplishing a surgical task. In some embodiments, the embolic material filter assembly is configured to, in the deployed configuration, interface with a patient's aorta and substantially block flow of embolic material through the patient's aorta past the embolic material filter assembly. In some embodiments, the treatment catheter is configured for deploying a prosthetic aortic valve.

The embolic material filter can be configured to be coupled with an embolic material extraction device. The embolic material extraction device can be operable to draw embolic material through the inner sheath lumen while the embolic material filter assembly is in the deployed configuration.

In some embodiments, the embolic material filter assembly includes an outer scaffold and an inner filter attached to the outer scaffold. The outer scaffold can be configured to radially expand into contact with a blood vessel along which embolic material is blocked from traversing. The inner filter can be configured to prevent emboli of greater than a particular size from passing through the inner filter. The outer scaffold can include distally extending loops of wires configured for atraumatic engagement of the blood vessel.

In some embodiments, a middle portion of the embolic material filter assembly has a middle portion external diameter in the deployed configuration. The distal end portion of the embolic material filter assembly has a distal end portion external diameter in the deployed configuration. In some embodiments, the middle portion external diameter is less than the distal end portion external diameter.

In many embodiments, the embolic material filter assembly includes an outer scaffold and an inner filter. The inner filter can be separated from the outer scaffold by an intervening annular space in the deployed configuration along a length of the inner filter.

In some embodiments, a proximal end portion of the outer scaffold is formed by proximally extending unbraided outer scaffold wire segments that are bonded to an outer surface of the inner sheath. Each of the unbraided outer scaffold wire segments can have a reduced diameter produced via electro-polishing.

In another aspect, an embolic material filter catheter includes an inner sheath, an embolic material filter assembly, a dilator assembly, and an introducer sheath. The inner sheath defines an inner sheath lumen. The embolic material filter assembly is coupled to a distal portion of the inner sheath. The embolic material filter assembly is reconfigurable from an insertion configuration to a deployed configuration. The insertion configuration accommodates insertion of the distal portion of the inner sheath and the embolic material filter assembly into a blood vessel of a patient to position the embolic material filter assembly downstream of a treatment site. In the deployed configuration, the embolic material filter assembly has an outer circumference configured to interface with the blood vessel. The dilator assembly is configured for advancement and retraction through the inner sheath lumen. The dilator assembly has a holding configuration and a non-holding configuration. The dilator assembly in the holding configuration restrains a distal end portion of the embolic material filter assembly in the insertion configuration. Reconfiguration of the dilator assembly from the holding configuration to the non-holding configuration releases the distal end portion of the embolic material filter assembly to accommodate reconfiguration of the embolic material filter assembly to the deployed configuration. The introducer sheath defines an introducer sheath lumen configured to accommodate advancement of the embolic material filter assembly, the inner sheath, and the dilator assembly. The introducer sheath includes a tapered distal end portion having a longitudinal length and an outer diameter that tapers distally from a proximal outer diameter at a proximal end of the tapered distal end portion down to a distal outer diameter at a distal end of the tapered distal end portion.

The tapered distal end portion of the introducer sheath can have any suitable configuration. For example, in exemplary embodiments, a diameter ratio of the distal outer diameter to the proximal outer diameter is in a range from 0.80 to 0.95. In exemplary embodiments, a length ratio of the longitudinal length to the distal outer diameter is in a range from 1.0 to 4.0.

In many embodiments, the distal end of the tapered distal end portion of the introducer sheath and the dilator assembly are configured to have an interference fit between the distal end of the tapered distal end portion of the introducer sheath and the dilator assembly. The tapered distal end portion of the introducer sheath and the dilator assembly can have any suitable configuration to produce the interference fit. For example, in some exemplary embodiments, an inner diameter of the distal end of the tapered distal end portion is in a range from 0.97 to 0.99 times an outer diameter of the dilator assembly.

In many embodiments, the tapered distal end portion of the introducer sheath includes a strain-relief feature configured to reduce circumferentially oriented strain in the distal end of the tapered distal end portion of the introducer sheath resulting from the interference fit between the distal end of the tapered distal end portion of the introducer sheath and the dilator assembly. The strain-relief feature can have any suitable configuration. For example, in some exemplary embodiments, the strain-relief feature includes a through-thickness slit that extends proximally from the distal end of the tapered distal end portion. In some other exemplary embodiments, the strain-relief feature includes a through-thickness slot that extends proximally from the distal end of the tapered distal end portion. In some embodiments, the tapered distal end portion includes an external surface having an atraumatic shape that extends from and surrounds the through-thickness slot.

For a fuller understanding of the nature and advantages of the present invention, reference should be made to the ensuing detailed description and accompanying drawings.

### BRIEF DESCRIPTION OF THE DRAWINGS

**FIG.** 1 shows an embolic material filter catheter in an insertion configuration for insertion into and advancement through the vasculature of a patient, in accordance with many embodiments.
**FIG. 2** shows an embolic material filter assembly of the embolic material filter catheter of FIG. 1 in an insertion configuration of the embolic material filter assembly.
FIG. 3 shows a first intermediate state of expansion of an embolic material filter assembly of the embolic material filter catheter of **FIG. 1****.**
**FIG. 4** shows a second intermediate state of expansion of the embolic material filter assembly of the embolic material filter catheter of **FIG. 1****.**
**FIG. 5** shows the embolic material filter assembly of the embolic material filter catheter of **FIG. 1** in a deployed configuration.
**FIG. 6** shows a close-up cross-sectional view of a distal end portion of a dilator assembly of the embolic material filter catheter of **FIG. 1** that is used to hold the distal end of the embolic material filter assembly in the insertion configuration and release the distal end of the embolic material filter assembly during deployment of the embolic material filter assembly.
**FIG. 7** shows the embolic material filter catheter of **FIG. 1** with the embolic material filter assembly fully deployed, the dilator assembly removed, and ready for insertion of a treatment catheter to perform a surgical task upstream of the deployed embolic material filter assembly.
**FIG. 8** shows the embolic material filter catheter of **FIG. 1** with the embolic material filter assembly fully deployed within a patient's aorta, the dilator assembly removed, and ready for insertion of a treatment catheter to perform a surgical task upstream of the deployed embolic material filter assembly.
**FIG. 9** shows the embolic material filter catheter of **FIG. 1** with the embolic material filter assembly fully deployed within a patient's aorta, the dilator assembly removed, and an aortic replacement valve deployment catheter inserted through the embolic material filter catheter and positioned to deploy an aortic replacement valve.
**FIG. 10** shows deployment of the aortic replacement valve from the delivery configuration of **FIG. 9** via expansion of an expandable member of the aortic replacement valve deployment catheter.
**FIG. 11** shows the embolic material filter catheter of **FIG. 1** with the embolic material filter assembly captured within an introducer sheath of the embolic material filter catheter.
**FIG. 12** is a simplified block diagram of acts of a method of deploying an embolic material capture filter assembly in a blood vessel, in accordance with many embodiments.
**FIG. 13** shows a side view of an embodiment of the embolic material filter assembly of the embolic material filter catheter of **FIG. 1****.**
**FIG. 14** shows a side cross-sectional view of the embodiment of the embolic material filter assembly of **FIG. 13****.**
**FIG. 15** shows a side cross-sectional view of an embodiment of the embolic material filter assembly of the embolic material filter catheter of **FIG. 1** that includes a pleated inner filter that accommodates axial contraction of an outer scaffold of the embolic material filter assembly during deployment.
**FIG. 16** is a picture of a prototype of the pleated inner filter of **FIG. 15****.**
**FIG. 17** shows a side cross-sectional view of an embodiment of the embolic material filter assembly of the embolic material filter catheter of **FIG. 1** that includes a helically-pleated inner filter that accommodates axial contraction of the outer scaffold of the embolic material filter assembly during deployment.
**FIG. 18** is an end-view picture of a prototype of the helically-pleated inner filter of **FIG. 17****.**
**FIG. 19** is a picture of an embodiment of the embolic material filter assembly of the embolic material filter catheter of **FIG. 1** that includes an outer scaffold and an inner filter distal end portion with a zig-zag shaped distal end that is aligned with and attached to a weave of the outer scaffold.
**FIG. 20** shows a side view of an embodiment of an embolic material filter assembly of the embolic material filter catheter of **FIG. 1** that includes an outer scaffold with proximal end wire segments with reduced wire diameter to minimize an outer diameter of the filter catheter at the connection between the outer scaffold and the inner sheath.
**FIG. 21** shows a side view of an embodiment of an embolic material filter assembly of the embolic material filter catheter of **FIG. 1** that includes an outer scaffold with unbraided proximal end wire segments with reduced wire diameter to minimize an outer diameter of the filter catheter at the connection between the outer scaffold and the inner sheath.
**FIG. 22** shows a side view of the dilator assembly of the embolic material filter catheter of **FIG. 1** extending distally from the introducer sheath of the embolic material filter catheter.
**FIG. 23** shows a side view of a tapered distal end portion of the introducer sheath of the embolic material filter catheter of **FIG. 1** in an unexpanded configuration.
**FIG. 24** shows a side view of the tapered distal end portion of the introducer sheath of the embolic material filter catheter of **FIG. 1** in an expanded configuration produced via radial expansion generated via interaction between the dilator assembly and the tapered distal end portion.
**FIG. 25** shows another side view of the tapered distal end portion of the introducer sheath of the embolic material filter catheter of **FIG. 1** in an expanded configuration produced via radial expansion generated via interaction between the dilator assembly and the tapered distal end portion.
**FIG. 26** shows a cross-sectional view BB defined in **FIG. 25** and a close-up view of the distal end of the introducer sheath interfaced with an outer surface recess in the dilator assembly.
**FIG. 27** shows a side-view of the distal end portion of the dilator assembly of **FIG. 25** and **FIG. 26****.**
**FIG. 28** shows a side-view of a longitudinal strain-relief slit in the tapered distal end portion of an embodiment of the introducer sheath.
**FIG. 29** shows a side-view of a longitudinal strain-relief slot in the tapered distal end portion of another embodiment of the introducer sheath.
**FIG. 30** shows a photograph of a prototype of the introducer sheath with a longitudinal strain-relief slot in the tapered distal end portion of another embodiment of the introducer sheath.

For a fuller understanding of the nature and advantages of the present invention, reference should be made to the ensuing detailed description and accompanying drawings.

### DETAILED DESCRIPTION

In the following description, various embodiments of the present invention will be described. For purposes of explanation, specific configurations and details are set forth in order to provide a thorough understanding of the embodiments. However, it will also be apparent to one skilled in the art that the present invention may be practiced without the specific details. Furthermore, well-known features may be omitted or simplified in order not to obscure the embodiment being described.

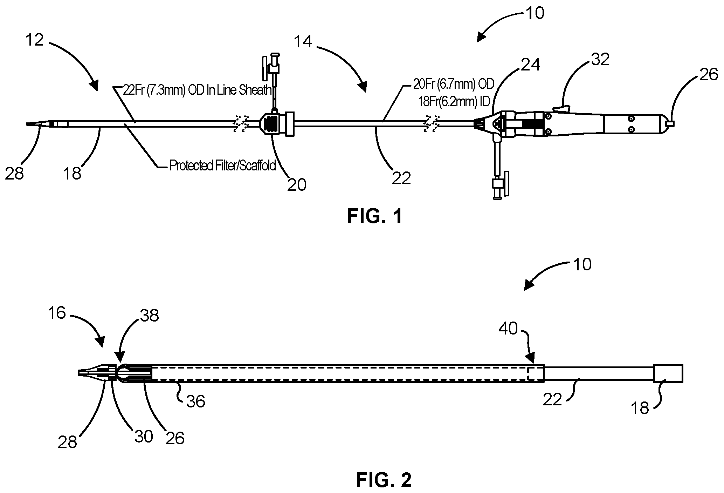

Referring now to the drawings, in which like reference numerals represent like parts throughout the several views, **FIG. 1** shows an embolic material filter catheter 10 in an insertion configuration for insertion into and advancement through the vasculature of a patient, in accordance with many embodiments. The filter catheter 10 includes an introducer sheath assembly 12, an inner sheath assembly 14, and a dilator assembly 16. The introducer sheath assembly 12 includes an introducer sheath 18 and an introducer sheath proximal end assembly 20 attached to the introducer sheath 18. The introducer sheath 18 is a flexible tube having an introducer sheath lumen extending through the introducer sheath 18. The introducer sheath proximal end assembly 20 remains external to the patient and can be used to distally advance the introducer sheath 18 through the blood vessel and proximally retract the introducer sheath 18 along and/or from the vasculature. The inner sheath assembly 14 includes an inner sheath 22, an inner sheath proximal end assembly 24 attached to the inner sheath 22, and an embolic material filter assembly 36 (shown in **FIG. 2**) attached to the distal end of the inner sheath 22. The inner sheath 22 is a flexible tube having an inner sheath lumen extending through the inner sheath 22. The inner sheath 22 is slidably disposed within the introducer sheath lumen of the introducer sheath 18. In many embodiments, the introducer sheath proximal assembly 20 includes a seal that interfaces with the outer surface of the inner sheath 22 to inhibit and/or prevent escape of bodily fluid (e.g., blood) from an annular space between the inner sheath 22 and the introducer sheath 18. The inner sheath proximal end assembly 24 remains external to the patient and can be used to distally advance the inner sheath 22 through the introducer sheath 18 and the vasculature and proximally retract the inner sheath 22 along and/or from the introducer sheath 18 and the vasculature. During initial advancement of the filter catheter 10 into and through the vasculature of the patient, the configuration shown in **FIG. 1** in which the embolic material filter assembly 36 is disposed within the introducer sheath lumen of the introducer sheath 18 can be employed. The inner sheath 22 can then be advanced distally relative to the introducer sheath 18 during a later stage of advancement of the embolic material filter assembly 22 through the vasculature.

The dilator assembly 16 is used to restrain the distal end of the filter assembly 36 in the insertion configuration of the filter assembly 36. The dilator assembly 16 includes a dilator shaft 26, a dilator distal end member 28, and a dilator pull wires 30. The dilator shaft 26 includes a lumen for each of the dilator pull wires 30. The dilator pull wires 30 are coupled to a pull wire actuation member 32 that is operable to retract the dilator pull wires 30 proximally relative to the dilator distal end member 28. The dilator distal end member 28 is attached to a distal end of the dilator shaft 26. The dilator shaft 26 extends through and is slidably disposed within the inner sheath lumen of the inner sheath 22. As described herein, the dilator distal end member 28 and the dilator pull wires 30 restrain the distal end portion of the embolic material filter assembly 36 when the filter assembly 36 is in the insertion configuration.

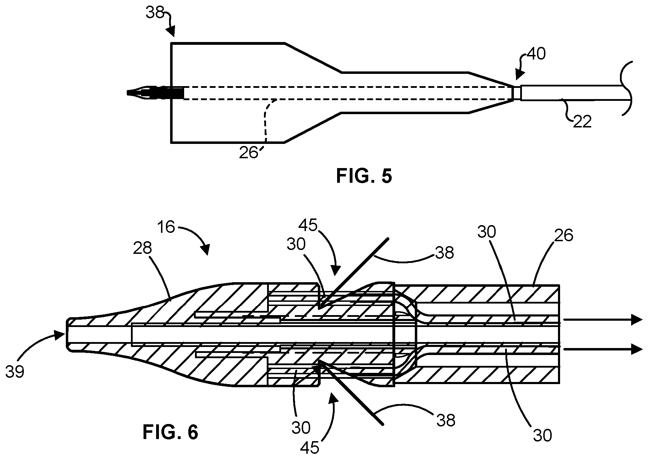

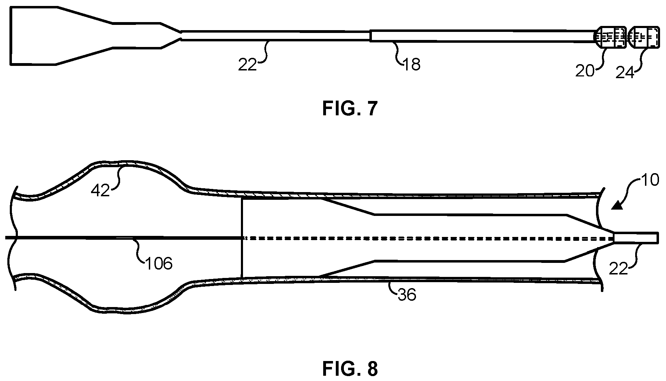

During deployment of the embolic material filter assembly 36, the pull wire actuation member 32 is operated to retract the dilator pull wires 30 proximally relative to the dilator distal end member 28 to release the distal end portion of the embolic material filter assembly 36 from engagement with the dilator distal end member 28 and the dilator pull wires 30, thereby allowing self-expansion of the distal end portion of the embolic material filter assembly 36 as described herein. In many embodiments, the embolic material filter catheter 10 is configured for deployment over a guidewire 106 (shown in **FIG. 8**). For example, the dilator assembly 16 can include a guide wire lumen 39 (shown in **FIG. 6**) that extends through the dilator assembly 16.

**FIG. 2** shows a distal portion of the embolic material filter catheter 10 in an insertion configuration. The illustrated portion of the catheter 10 includes a distal end portion of the introducer sheath 18, a distal end portion of the inner sheath 22 extending distally beyond the lumen of the introducer sheath 18, the embolic material filter assembly 36 in a collapsed insertion configuration, a distal end portion of the dilator shaft 26, the dilator distal end member 28, and the dilator pull wires 30. In the illustrated insertion configuration, suture loops 38 are attached to a distal end portion 38 of the embolic material filter assembly 36. Each of the suture loops 38 passes through a respective set of distal end loops of an outer scaffold 44 of the filter assembly 36 and passes through a respective passage formed between a respective one of the dilator pull wires 30 and the dilator distal end member 28 in the insertion configuration. With the suture loops 38 restrained via engagement with the dilator distal end member 28 and the dilator wires 30 and a proximal end portion 40 of the embolic material filter assembly 36 attached to the distal end of the inner sheath 22, a suitable position of the inner sheath 22 relative to the dilator distal end member 28 can be maintained to retain the embolic material filter assembly 36 in the collapsed insertion configuration. For example, in some embodiments, the embolic material filter assembly 36, in the illustrated collapsed insertion configuration, is under axial tension induced via opposed axial forces applied to the embolic material filter assembly 36 by the dilator assembly 16 and the inner sheath 22. In some embodiments, the embolic material filter assembly 36 conforms at least in part to an outer surface of the dilator shaft 26 from the proximal end portion of the embolic material filter assembly 36 to the distal end portion of the embolic material filter assembly 36 when the embolic material filter assembly 36 is in the insertion configuration. In many embodiments, the dilator distal end member 28 as an atraumatic shape to protect the vasculature during distal advancement of the catheter 10 through the vasculature. In the illustrated configuration, the inner sheath 22 is positioned relative to the introducer sheath 18 so that the proximal end of the embolic material filter assembly 36 is disposed distal to the distal end of the introducer sheath 18 and maintained in the collapsed insertion configuration without the use of a sheath surrounding the embolic material filter assembly 36.

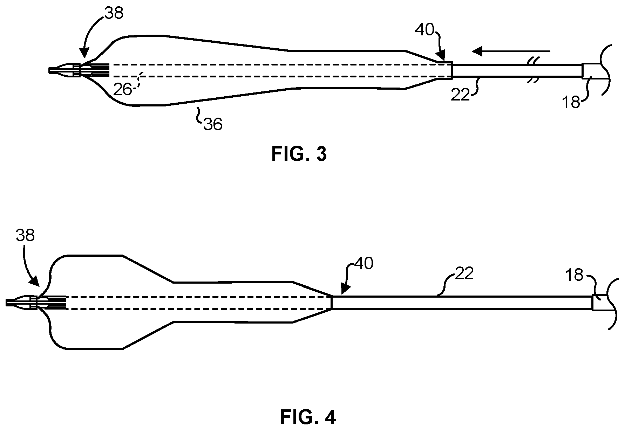

**FIG. 3** and **FIG. 4** illustrate progressive expansion of the embolic material filter assembly 36 from the insertion configuration shown in **FIG. 2** to the intermediate deployment configuration shown in **FIG. 4** via distal advancement of the inner sheath 22 relative to the dilator assembly 16 while the suture loops 38 of the embolic material filter assembly 36 remain restrained by the dilator distal end member 28 and the dilator pull wires 30. As shown, a middle portion of the embolic material filter assembly 36 expands radially from the insertion configuration to the intermediate deployment configuration.

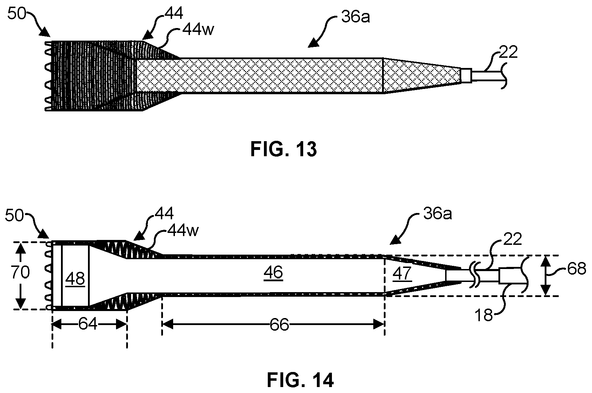

Upon release of the suture loops 38 via retraction of the dilator pull wires 30, the distal end of the embolic material filter assembly 36 self-expands from the intermediate deployment configuration to the deployed configuration shown in **FIG. 5****.** In many embodiments, the embolic material filter assembly 36 includes an outer scaffold 44 (shown in **FIG. 13** and **FIG. 14**) that is configured to self-expand from the insertion configuration to the deployed configuration in which an outer surface of the embolic material filter assembly 36 engages a blood vessel.

**FIG. 6** shows a close-up end view of the distal portion of the dilator assembly 16 in the intermediate deployed configuration of the embolic material filter catheter 10 (shown in **FIG. 4**). In both the insertion configuration and the intermediate deployed configuration, each of the suture loops 38 of the embolic material filter assembly 36 is trapped within a proximal recess 45 in the dilator distal end member 28 and retained in the proximal recess 45 via a respective one of the dilator pull wires 30. Any suitable number of the dilator pull wires 30 and corresponding number of the suture loops 38 can be employed, such as two, three, four, five, six, seven, or more. For example, in the illustrated embodiment, there are six circumferentially-distributed dilator pull wires 30 and corresponding six circumferentially-distributed suture loops 38. Each of the suture loops 38 can be passed through a suitable number of end loops of the outer scaffold 44. For example, the outer scaffold 44 can have 24 end loops and each six suture loops 38 can pass through a respective set of four of the end loops of the outer scaffold 44. By passing each of the suture loops 38 through a respective set of two or more end loops of the outer scaffold 44, tension within each of the suture loops in the insertion configuration cause the suture loops 38 to function like draw strings to bring the respective set of end loops of the outer scaffold 44 together, thereby enhancing consolidation of the end loops of the outer scaffold 44 around the dilator shaft 26 in the collapsed insertion configuration of the embolic material filter assembly 36. Proximal retraction of the dilator pull wires 30 is used to release the suture loops 38, thereby releasing the distal end of the embolic material filter assembly 36.

From the configuration shown in **FIG. 5****,** the dilator assembly 16 can be removed through the inner sheath lumen of the inner sheath 22 to make the inner sheath lumen available for insertion of a treatment catheter through inner sheath lumen of the inner sheath 22 to perform a surgical task upstream of the embolic material filter assembly 36 in the fully deployed configuration. **FIG. 7** shows the embolic material filter catheter 10 with the embolic material filter assembly 36 in the deployed configuration with the dilator assembly 16 removed. Because the inner sheath 22 is advanced distally relative to the introducer sheath assembly 12 to deploy the embolic material filter assembly 36, the inner sheath proximal end assembly 24 can be in close proximity with the introducer sheath proximal end assembly 20 when the embolic material filter assembly 36 is in the deployed configuration, thereby enabling the use of existing length treatment catheters.

In many embodiments, the embolic material filter assembly 36 includes the outer scaffold 44 and an inner filter 46 attached to the outer scaffold. The outer scaffold 44 can include one or more members that radially expand into contact with the wall of a vessel along which embolic material is blocked from traversing. The inner filter 46 can include a filtering device or filtering membrane configured to prevent emboli of greater than a particular size from passing through the filtering device or the filtering membrane. For example, to capture emboli greater than or equal to 200 microns in size, the inner filter 46 can have apertures of 200 microns or less in size. To provide for a suitable pressure drop across the inner filter 46 in use, the inner filter 46 can have apertures between a suitable minimum size (e.g., 50 microns) and a suitable maximum size (e.g., 200 microns) for capturing emboli greater than or equal to 200 microns in size. In some embodiments, the inner filter 46 has 140 micron apertures to provide a suitable balance between size of emboli captured and a suitable pressure drop across the inner filter 46 in use. The outer scaffold 44 is configured to provide a framework and stability for the inner filter 46 to function.

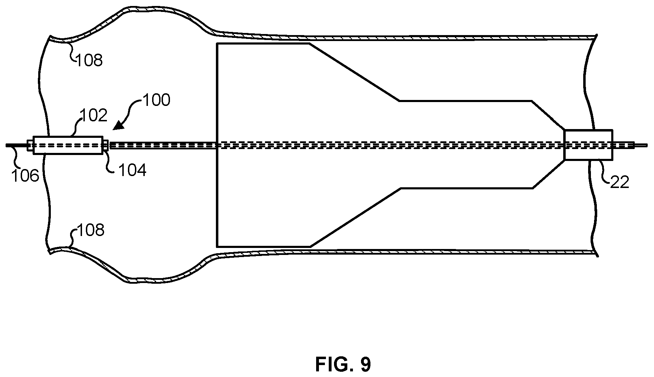

The embolic material filter catheter 10 can be configured for use in any suitable blood vessel and for use with any suitable treatment catheter. For example, **FIG. 8** shows an embodiment of the embolic material filter catheter 10 deployed within a patient's aorta 42 to capture embolic material liberated during implantation of a prosthetic aortic valve. **FIG. 9** shows the embolic material filter catheter 10 with an aortic replacement valve deployment catheter 100 inserted through the inner sheath lumen of the inner sheath 22 and positioned to deploy an aortic replacement valve 102. The replacement valve deployment catheter 100 includes an expandable member 104 that is expanded to deploy the valve 102. **FIG. 9** also illustrates the expandable member 104 (and the prosthetic valve 102 mounted to the expandable member 104 in a collapsed configuration) positioned for implantation of the prosthetic valve 102 after being advance along a guide wire 106 and through the inner sheath lumen of the inner sheath 22. With the embolic material filter assembly 36 deployed downstream of the patient's native aortic valve 108, the embolic material filter assembly 36 is positioned to capture embolic material released during deployment of the prosthetic aortic valve 102.

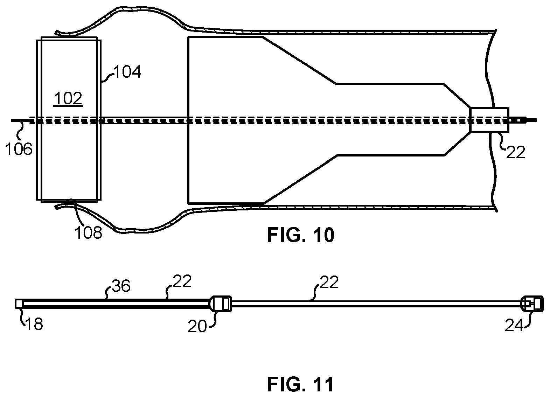

**FIG. 10** illustrates deployment of the prosthetic valve 102 at the implantation site via expansion of the expandable member 104. The expansion of the expandable member 104 expands the prosthetic aortic valve 102 into its deployed configuration covering the native aortic valve 108. The expandable member 104 can be expanded during rapid pacing of the patient's heart. Embolic material released during deployment of the prosthetic aortic valve 102 is captured by the embolic material filter assembly 36. In some embodiments, the embolic material filter catheter 10 can be fluidly coupled with an external embolic material removal device operable to remove embolic material gathered by the embolic material filter assembly 36 from the patient. Following implantation of the prosthetic aortic valve 102, the replacement valve deployment catheter 100 can be removed from the embolic material protection catheter 10 via proximal retraction through the inner sheath lumen of the inner sheath 22

In many embodiments, the embolic material filter catheter 10 can be reconfigured to capture the embolic material filter assembly 36 prior to withdrawal of the embolic material filter catheter 10 from the patient. For example, in the illustrated embodiment, the embolic material filter catheter 10 can be reconfigured from the configuration shown in **FIG. 8** in which the embolic material filter assembly 36 is in the deployed configuration to the configuration shown in **FIG. 11** in which the embolic material filter assembly is in the captured configuration via proximal retraction of the inner sheath assembly 14 relative to the introducer sheath assembly 12, thereby pulling the embolic material filter assembly 36 into the introducer sheath lumen of the introducer sheath 18.

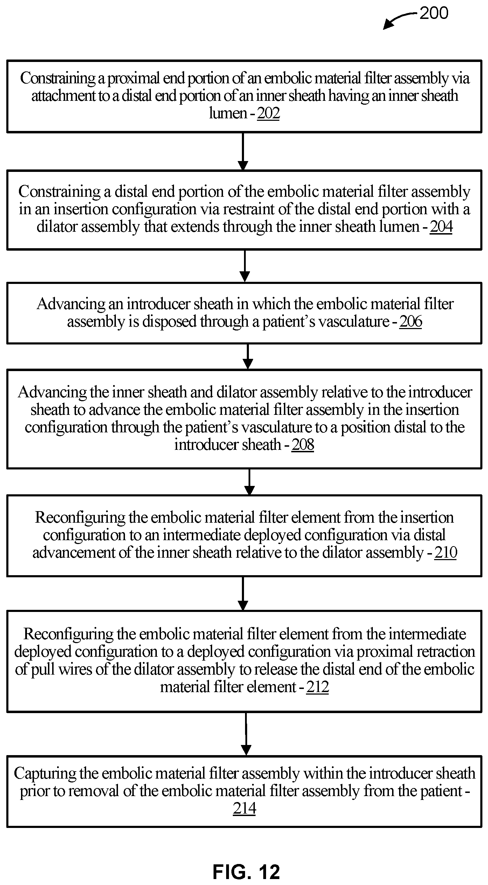

**FIG. 12** is a simplified block diagram of acts of a method 200 of deploying an embolic material filter assembly in a blood vessel, in accordance with many embodiments. The method 200 can be practiced using any suitable device or devices, including the embolic material filter catheter 10 described herein. The method 200 can be used to provide embolic protection in conjunction with any suitable treatment, including the treatments indicated herein.

The method 200 includes constraining a proximal end portion of an embolic material filter assembly via attachment to a distal end portion of an inner sheath having an inner sheath lumen (act 202). For example, as illustrated in **FIG. 2****,** the proximal end 40 of the embolic material filter assembly 36 is attached to the distal end of the inner sheath 22 in the embolic material filter catheter 10.

The method 200 further includes constraining a distal end portion of the embolic material filter assembly in an insertion configuration and an intermediate deployment configuration of the embolic material filter assembly via restraint of the distal end portion with a dilator assembly that extends through the inner sheath lumen (act 204). For example, in the insertion configuration illustrated in **FIG. 2** and the intermediate deployment configuration illustrated in **FIG. 4****,** the suture loops 38 of the embolic material filter assembly 38 are restrained to the dilator distal end member 28 via the dilator pull wires 30.

The method 200 further includes advancing an introducer sheath in which the embolic material filter assembly is disposed through a patient's vasculature (act 206). For example, the configuration of the filter catheter 10 shown in **FIG. 1** can be used during introduction and initial advancement of the embolic material filter assembly 36 through a patient's vasculature.

The method 200 further includes advancing the inner sheath and the dilator assembly relative to the introducer sheath to advance the embolic material filter assembly in the insertion configuration through the patient's vasculature to a position distal to the introducer sheath (act 208). For example, act 208 can be accomplished via advancing the inner sheath assembly 14 distally relative to the introducer sheath assembly 12 to reconfigure the filter catheter 10 from the insertion configuration shown in **FIG. 1** to the insertion configuration shown in **FIG. 2****.**

The method 200 further includes reconfiguring the embolic material filter assembly from the insertion configuration to the intermediate deployment configuration by expanding a middle portion of the embolic material filter assembly disposed between the proximal end portion of the embolic material filter assembly and the distal end portion of the embolic material filter assembly via distal advancement of the inner sheath toward the distal end portion of the embolic material filter assembly constrained by the dilator assembly (act 210). For example, act 210 can be accomplished via reconfiguring the embolic material filter catheter 10 from the insertion configuration illustrated in **FIG. 2** to the intermediate deployment configuration illustrated in **FIG. 4** as described herein.

The method 200 further includes reconfiguring the embolic material filter assembly from the intermediate deployment configuration to the deployed configuration via reconfiguration of the dilator assembly to release the distal end portion of the embolic material filter assembly from engagement with the dilator assembly and self-expansion of the distal end portion of the embolic material filter assembly (act 212). For example, act 212 can be accomplished via reconfiguring the embolic material filter catheter 10 from the intermediate deployment configuration illustrated in **FIG. 4** to the fully deployed configuration illustrated in **FIG. 5** as described herein.

The method 200 further includes capturing the embolic material filter assembly within the introducer sheath prior to proximal retraction of the embolic material filter assembly from the patient's vasculature (act 214). For example, act 214 can be accomplished via retracting the inner sheath assembly 14 proximally relative to the introducer sheath assembly 12 to reconfigure the filter catheter 10 from the deployed configuration shown in **FIG. 7** to the captured configuration shown in **FIG. 11****.**

In many embodiments, the embolic material filter assembly 36 has a flexibility so as to have a deployed shape in which the distal end of the embolic material filter assembly 36 conforms to the inner surface of the blood vessel (e.g., the aorta 42) and conforms to the shape of the blood vessel. For example, in many embodiments, the embolic material filter assembly 36 is configured to conform to the shape (e.g., curvature) of the blood vessel between the distal end portion of the embolic material filter assembly 36 and the proximal end portion 40 (e.g., to the curvature of the aorta 42).

### Embolic Material Filter Assembly Configurations

The embolic material filter assembly 36 can have any suitable configuration. For example, **FIG. 13** shows a side view of an embodiment 36a of the embolic material filter assembly 36 in the deployed configuration. **FIG. 14** shows a side cross-sectional view of the embodiment 36a of the embolic material filter assembly 36 in the deployed configuration. The embodiment 36a of the embolic material filter assembly 36 includes an outer scaffold 44 and an inner filter 46.

The outer scaffold 44 includes one or more inter-braided helically-shaped lengths of a suitable wire 44w (e.g., 0.006 to 0.010 inch diameter wire made from a suitable nickel-titanium shape-memory alloy). In many embodiments, strands of the wire 44w are alternately woven (e.g., passed over and then under) crossing portions of the strands of the wire 44w. From the insertion configuration shown in **FIG. 2** to the fully deployed configuration shown in **FIG. 13** and **FIG. 14****,** a pitch between adjacent locations on the wire 44w decreases from a suitable initial pitch to a suitable deployed pitch (DP) and the diameter of the outer scaffold 44 increases from a suitable insertion configuration diameter to a suitable fully deployed configuration diameter, thereby producing a corresponding decrease in the length of the outer scaffold 44. The wire 44w of the outer scaffold 44 has a shape memory such that the outer scaffold 44 automatically reconfigures from the insertion configuration to the fully deployed configuration in response to the distal advancement of the inner sheath 22 and the release of the distal end portion of the first configuration 36a of the embolic material filter assembly 36. Upon retraction of the first configuration 36a of the embolic material filter assembly 36 into the introducer sheath 18, the pitch between adjacent locations on the wire 44w increases and the diameter of the outer scaffold 44 decreases, thereby producing a corresponding increase in the length of the outer scaffold 44. In the illustrated embodiment, the outer scaffold 44 has an atraumatic leading edge 50 formed by loops of the wire 44w. In some embodiments, the outer scaffold 44 includes capture loops that are engaged with the suture loops 38, which are restrained in contact with the dilator distal end member 28 via the dilator pull wires 30 while the embolic material filter assembly 36 is in the insertion configuration. In some embodiments, the maximum expanded diameter of the embolic material filter assembly 36 is sized to ensure engagement with a patient's aortic arch.

In many embodiments, the inner filter 46 has a suitable porosity that provides for capture of embolic material by the inner filter 46 while accommodating blood flow through the inner filter 46. The inner filter 46 can be made from any suitable material. For example, in some embodiments, the inner filter 46 includes a helically-braided polyethylene terephthalate (PET) filter. In some embodiments, the inner filter 46 includes a helically-braided polymer filter made from a suitable polymer yarn such as ultra-high-molecular-weight polyethylene (UHMWPE), PET, nylon, polypropylene, polytetrafluoroethylene (PTFE), and liquid crystal polymer (LCP). In some embodiments, the inner filter 46 includes a laser cut polymer filter made from a suitable polymer material (e.g., elastomeric materials such as silicones, polyurethanes and co-polymers). In some embodiments, the inner filter 46 includes a woven textile filter with a diameter less than or equal to the braided outer scaffold 44 with target porosity to capture embolic material and allow for blood flow through the inner filter 46. Such a woven textile filter can be made from a suitable polymer yarn such as UHMWPE, PET, nylon, polypropylene, PTFE, and LCP. The inner filter 46 can have any suitable configuration. For example, in many embodiments, the inner filter 46 can have an outer diameter in the fully deployed configuration less than or equal to the inner diameter of the outer scaffold 44 in the fully deployed configuration. In many embodiments, the inner filter 46 has a longitudinal length and/or longitudinal flexibility that accommodates the change in length of the outer scaffold 44 between the insertion configuration and the fully deployed configuration.

The inner filter 46 can have any suitable configuration. For example, the inner filter 46 can includes a non-pleated distal end member 48 made from nylon and a pleated main body member that is attached to the distal end member 48 and formed from a suitable polyester material in which the pleats can be formed via heat setting.

In the deployed configuration, the embolic material filter assembly 36a has a stepped outer diametrical profile configured to enhance deployment from the insertion configuration to the deployed configuration by substantially isolating contact between the outer scaffold 44 and the blood vessel to a distal end portion 64 of the embolic material filter assembly 36a. In the deployed configuration, a middle portion 66 of the embolic material filter assembly 36a has a middle portion external diameter 68, and the distal end portion 64 has a distal end portion external diameter 70. In many embodiments, the distal end portion external diameter 70 is sized to provide a suitable amount of engagement with a target blood vessel. For example, the distal end portion external diameter 70 can be sized so that, in the deployed configuration, the distal end portion external diameter 70 would be a suitable increment larger than the inner diameter of the target blood vessel (e.g., aorta) so that the distal end portion 64 would exert a suitable interface pressure onto the target blood vessel when deployed in the target blood vessel. In many embodiments, the middle portion external diameter 68 is a suitably smaller than the inner diameter of the target blood vessel so that there is an annular clearance between the middle portion 66 and the inner wall of the target blood vessel to accommodate lengthwise contraction of the embolic material filter assembly 36a during deployment from the insertion configuration to the deployed configuration by substantially isolating contact between the outer scaffold 44 and the blood vessel to the distal end portion 64. The annular clearance between the middle portion 66 and the inner wall of the target blood vessel serves to avoid interaction between the portion of the embolic material filter assembly 36a proximal to the distal end portion 64 that might inhibit the contraction of the embolic material filter assembly 36a during deployment from the insertion configuration to the deployed configuration. The middle portion external diameter 68, in the deployed configuration, can be any suitable amount smaller than the inner diameter of the target blood vessel or the distal end portion external diameter 70. For example, in the illustrated embodiment, the middle portion external diameter 68 is about 50 percent of the distal end portion external diameter 70.

The illustrated embodiment 36a of the embolic material filter assembly 36, the inner filter 46 includes a proximal portion 47 that connects to the distal end of the inner sheath 22. In many embodiments, the proximal portion 47 has a shape (e.g., conical) that provides for a smooth transition to the distal end of the inner sheath 22. The proximal portion 47 can be attached to the inner sheath 22 using any suitable approach. For example, a proximal end portion of the proximal portion 47 can be bonded to the outer surface of the distal end portion of the inner sheath 22.

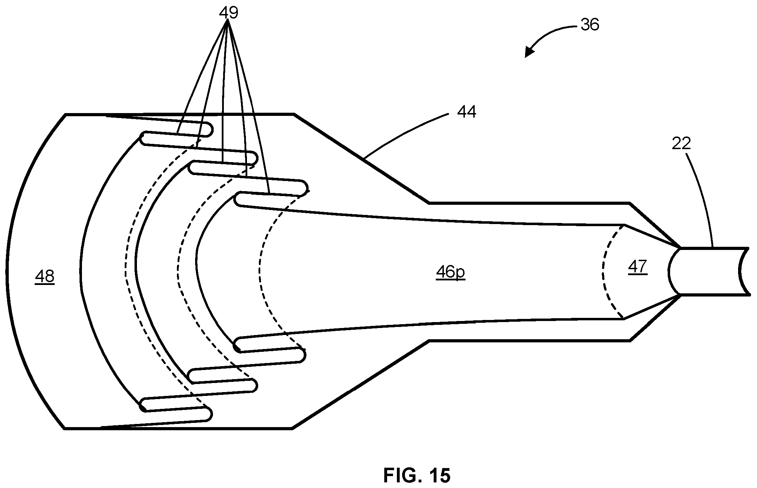

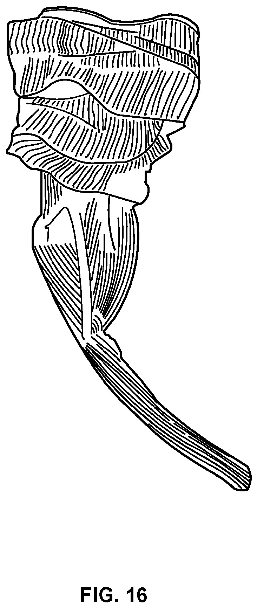

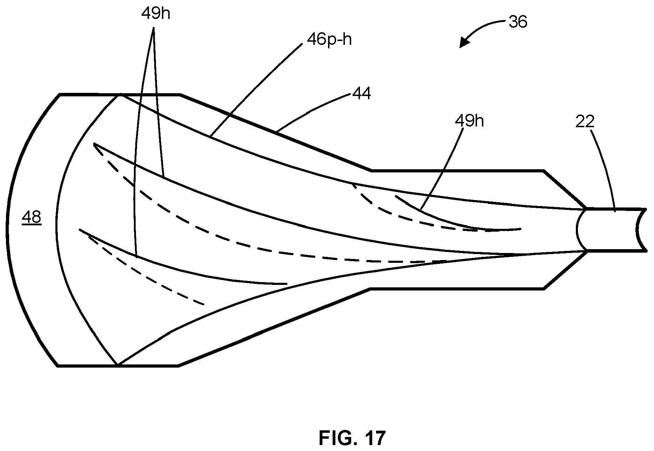

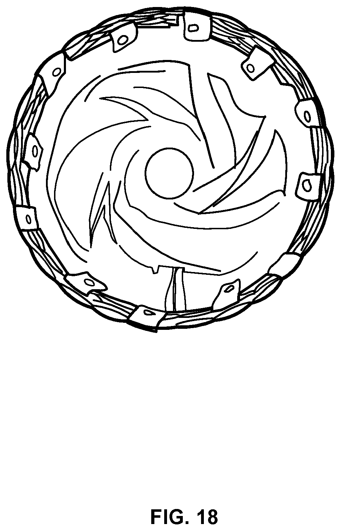

In some embodiments of the embolic material filter assembly 36, the inner filter 46 has pleats in the deployed configuration that accommodate contraction of the outer scaffold 44 during deployment of the embolic material filter assembly 36 from the insertion configuration to the deployed configuration. **FIG. 15** shows a side cross-sectional view of an embodiment of the embolic material filter assembly 36 that includes an inner filter 46p with pleats 49 in the deployed configuration as illustrated. The inner filter 46p can be configured to have the pleats 49 in the deployed configuration using any suitable approach. For example, the pleats 49 can be formed in the inner filter 46p using heat setting such that, in the absence of axial tension applied to the inner filter 46p by the outer scaffold 44 in the insertion configuration, the inner filter 46p has an unrestrained configuration that includes the pleats 49. **FIG. 16** is a picture of a prototype of the pleated inner filter 46p in the unrestrained configuration. **FIG. 17** shows a side cross-sectional view of an embodiment of the embolic material filter assembly 36 that includes an inner filter 46p-h with helical pleats 49h in the deployed configuration as illustrated. The inner filter 46p-h can be configured to have the helical pleats 49h in the deployed configuration using any suitable approach. For example, the helical pleats 49h can be formed in the inner filter 46p-h using heat setting such that, in the absence of axial tension applied to the inner filter 46p-h by the outer scaffold 44 in the insertion configuration, the inner filter 46p-h has an unrestrained configuration that includes the helical pleats 49h. **FIG. 18** is a picture of a prototype of the helically-pleated inner filter 46p-h in the deployed configuration of the embolic material filter assembly 36.

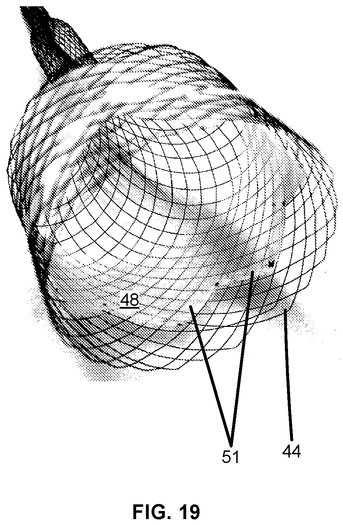

**FIG. 19** is a picture of an embodiment of the embolic material filter assembly 36 that includes the outer scaffold 44 and an embodiment of the inner filter 46 with a zig-zag shaped distal end 51. The zig-zag shaped distal end 51 is aligned with and woven into the braid of the outer scaffold 44 to secure the distal end 51 to the outer scaffold 44 so as to inhibit passage of emboli around the inner filter 46 and to minimize the resulting combined thickness of the embolic material filter assembly 36 at the distal end 51 of the inner filter 46. In some embodiments, the inner filter 46 includes a non-pleated distal end member 48 that includes the distal end 51, and further includes a pleated member that attached to the proximal end of the non-pleated distal end member 48 and extends to the inner sheath 22.

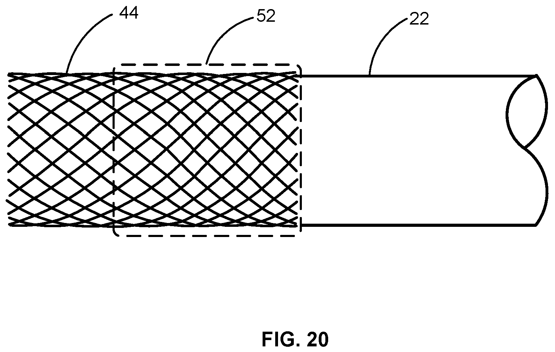

**FIG. 20** shows a close-up side view of an embodiment of the embolic material filter catheter 10 in which the proximal end of the outer scaffold 44 has wire segments 52 at the proximal end that are bonded to the outer surface of the inner sheath 22. The wire segments 52 are processed to reduce the thickness of the wire segments 52 relative to the wire segments of the outer scaffold 44 distal to the distal end of the inner sheath 22 to reduce the diameter of the inner sheath assembly 14 at the proximal end 40 of the embolic material filter assembly 36a bonded to the distal end of the inner sheath 22. For example, in some embodiments, the outer scaffold 44 is formed of braided 0.009 inch diameter wires and the proximal end segments that form the wire segments 52 are electro-polished down to a reduced diameter (e.g., 0.0055 to 0.006 inch).

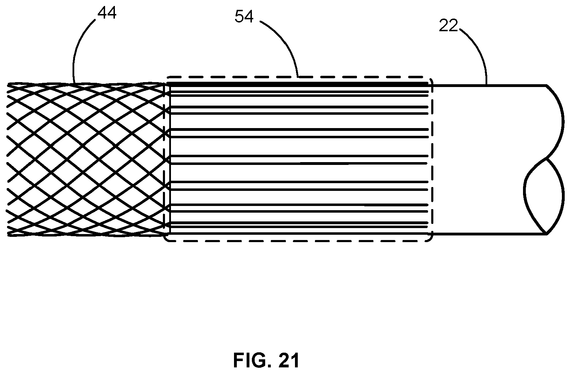

**FIG. 21** shows a close-up side view of an embodiment of the embolic material filter catheter 10 in which the proximal end of the outer scaffold 44 has non-braided wire segments 54 that are bonded to the outer surface of the inner sheath 22. While the non-braided wire segments 54 are arranged parallel to each in the embodiment shown in **FIG. 21****,** any suitable routing of the non-braided wire segments 54 can be employed. The non-braided wire segments 54 do not cross over or under each other, thereby providing for reduced thickness of the proximal portion of the outer scaffold 44 bonded to the inner sheath 22 due to the lack of stacking of the non-braided wire segments 54 at crossing points. Additionally, in some embodiments, the non-braided wire segments 54 are processed to reduce the thickness of the non-braided wire segments 54 relative to the wire segments of the outer scaffold 44 distal to the distal end of the inner sheath 22 to further reduce the diameter of the inner sheath assembly 14 at the proximal end 40 of the embolic material filter assembly 36a bonded to the distal end of the inner sheath 22. For example, in some embodiments, the outer scaffold 44 is formed of braided 0.009 inch diameter wires and proximal end segments that form the non-braided wire segments 54 are electro-polished down to a reduced diameter (e.g., 0.0055 to 0.006 inch).

### Introducer Sheath Tapered Distal End

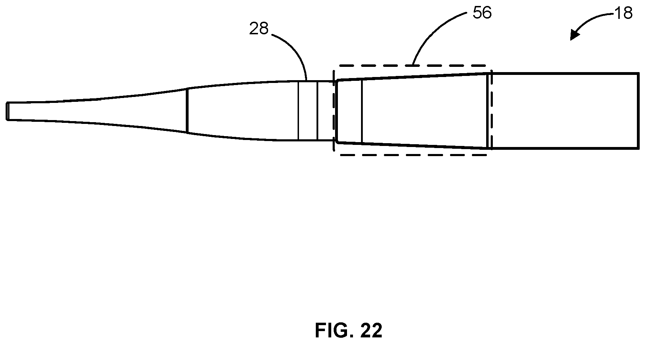

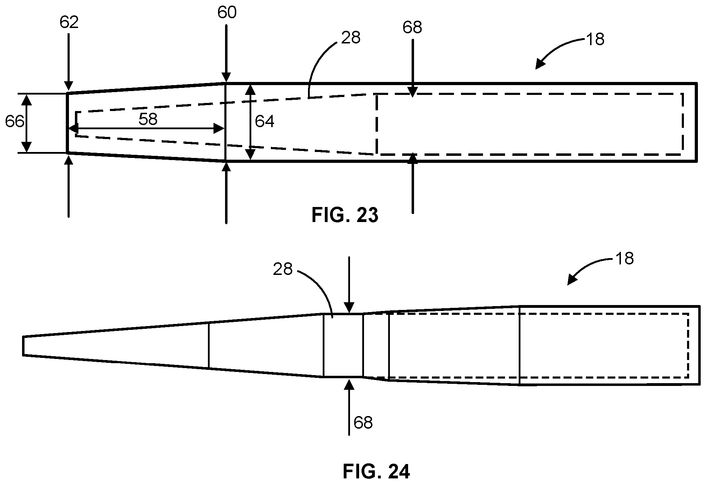

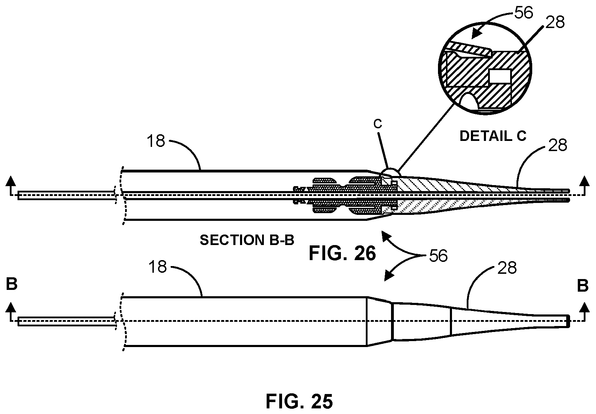

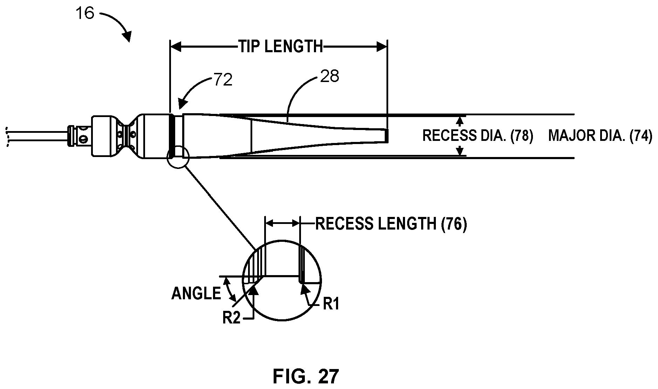

**FIG. 22****,** **FIG. 23,** and **FIG. 24** illustrate a tapered distal end portion 56 of the introducer sheath 18. The tapered distal end portion 56 has a longitudinal length 58, an outer diameter that tapers distally from a proximal outer diameter 60 at the proximal end of the tapered distal end portion 56 down to a distal outer diameter 62 at the distal end of the distal end portion 56. The distal outer diameter 62 can be less than the proximal outer diameter 60 by any suitable length. For example, in many embodiments, a ratio of the distal outer diameter 62 to the proximal outer diameter 60 is in a range between 0.80 and 0.95. The distal end portion 56 can have any suitable longitudinal length 58. For example, in some embodiments, a ratio of the longitudinal length 58 to the distal outer diameter 62 is in a range from 1.0 to 4.0. The tapered distal portion 56 has an inner diameter that tapers distally from a proximal inner diameter 64 at the proximal end down to a distal inner diameter 66 at the distal end. In many embodiments, the distal inner diameter 66 is equal to or less than a maximum outer diameter 68 of the dilator distal end member 28 so that the inner surface of the distal end portion 56 at the distal end of the distal end portion 56 conforms to the outer surface of the dilator distal end member 28 in the insertion configuration of the embolic material filter catheter 10 shown in **FIG. 1****,** **FIG. 22****,** and **FIG. 24****.** In many embodiments, the distal inner diameter 66 is less than the maximum outer diameter 68 of the dilator distal end member 28 by a suitable amount to produce a suitable interference fit between the introducer sheath 18 and the dilator distal end member 28 at the distal end of the tapered distal end portion 56. For example, in some embodiments, the distal inner diameter 66 is 0.98 times the outer diameter 68. In the insertion configuration shown in **FIG. 1****,** **FIG. 22****,** and **FIG. 24****,** the combination of the dilator distal end member 28 and the tapered distal end portion 56 of the introducer sheath 18 provides a smoothly tapered outer profile shape that serves to reduce tissue trauma induced during advancement of the embolic material filter catheter 10 through a patient's vasculature. **FIG. 25** shows another side view of the tapered distal end portion 56 of the introducer sheath 18 of the embolic material filter catheter of 10 in an expanded configuration produced via radial expansion generated via interaction between the dilator distal end member 28 and the tapered distal end portion 56 of the introducer sheath 18. **FIG. 26** shows a cross-sectional view BB defined in **FIG. 25** and a close-up view of the distal end of the tapered distal end portion 56 interfaced with an outer surface annular recess 72 in the dilator distal end member 28. **FIG. 27** shows a side-view of the distal end portion of the dilator assembly 16 including the dilator distal end member 28. The outer surface annular recess 72 is configured to receive and accommodate the distal end of the tapered distal end portion 56 (as shown in DETAIL C of **FIG. 26**) to shield the distal end of the tapered distal end portion 56 from frontal contact with tissue during advancement of the embolic material filter catheter 10 within a patient. The outer surface annular recess 72 can have any suitable configuration. For example, in the illustrated embodiment, dilator distal end member 28 has a major diameter 74 of 0.236 inches and the outer surface annular recess 72 has a recess length 76 of 0.05 inches and a recess diameter 78 of 0.218 inches, thereby producing a recess depth of 0.009 inches.

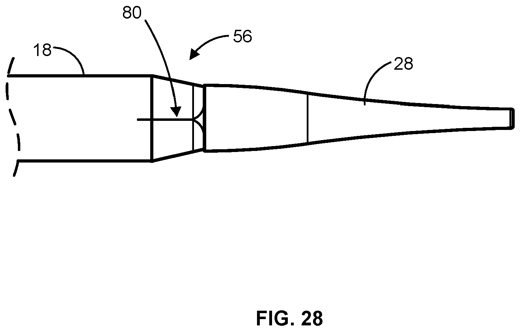

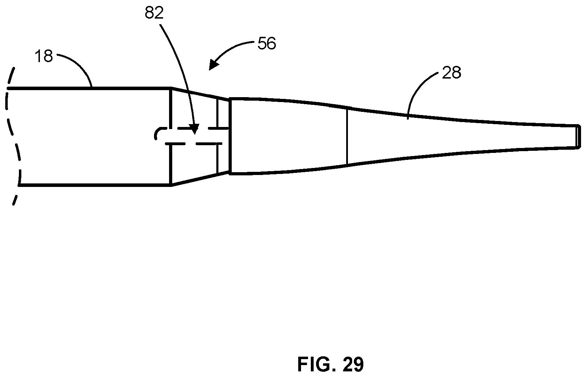

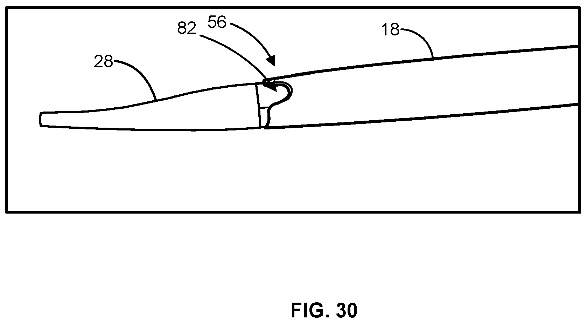

In some embodiments, the distal end portion of the introducer sheath 18 includes a strain-relief feature configured to increase compliance to accommodate diametrical expansion of the distal end portion of the introducer sheath 18 and thereby reduce longitudinal frictional forces during relative longitudinal movement between the introducer sheath 18 and the dilator assembly 16. In the embodiment illustrated in **FIG. 28****,** the strain-relief feature takes the form of a longitudinal strain-relief slit 80 in the tapered distal end portion 56 of an embodiment of the introducer sheath 18. In the embodiment illustrated in **FIG. 29****,** the strain-relief feature takes the form of a longitudinal strain-relief slot 82 in the tapered distal end portion 56 of an embodiment of the introducer sheath 18. **FIG. 30** shows a photograph of a prototype of the introducer sheath with a longitudinal strain-relief slot 82 in the tapered distal end portion 56 of an embodiment of the introducer sheath 18. During diametrical expansion of the distal end of the tapered distal end portion 56, the longitudinal strain-relief feature 80, 82 expands circumferentially, thereby reducing induced circumferential strain in the distal end portion of the tapered distal end portion 56 and therefore reducing radial interface forces and associated frictional forces between the distal end of the tapered distal end portion 56 and the dilator distal member 28. As shown in **FIG. 30****,** the prototype of the introducer sheath 18 includes an external surface having an atraumatic contoured shape that extends from and surrounds the longitudinal strain-relief slot 82. While the distal end portion of the introducer sheath 56 is illustrated with one longitudinal strain-relief feature 80, 82, any suitable number (e.g., 1, 2, 3, 4, or more) and length(s) of the strain-relief features 80, 82 can be included in the distal end portion of the introducer sheath 18.

The devices and methods described herein are expected to produce substantial benefits in the way of substantially increased safety and efficacy of surgical treatments with a high likelihood of generation of embolic material, such as aortic valve replacement. As a result, such surgical treatments may be performed on a substantially increased number of patients with improve outcomes and reduce recovery times. Specifically, there will be less embolic material conveyed within the circulation system, thereby lowering the incidence of clinical stroke, subclinical stroke, silent cerebral embolization, renal embolization, mesenteric embolization, and peripheral embolization and each of the associated clinical syndromes.

The embolic material filter catheter 10 is suitable for use in procedures involving covered or uncovered stenting of arteries for capture and extraction of embolic material that may be liberated during their implantation for the treatment of aneurysms, dissections, stenosis or thrombus. The embolic material filter catheter 10 is suitable for prevention of injury resulting from embolic events occurring during balloon aortic valvuloplasty. The embolic material filter catheter 10 is suitable for prevention of tissue injury resulting from the performance of mitral balloon valvuloplasty or replacement. In the case of mitral procedures, the embolic protection provided by the embolic material filter catheter 10 may be separate from a delivery catheter. In this situation there may be a separate transvenous or transapical implantation system of sheaths and catheters for valve delivery and deployment and the embolic material filter catheter 10 can be deployed in the ascending aorta for capture and elimination of the material liberated from the mitral valve manipulation .

While the invention is susceptible to various modifications and alternative constructions, certain illustrated embodiments thereof are shown in the drawings and have been described above in detail. It should be understood, however, that there is no intention to limit the invention to the specific form or forms disclosed, but on the contrary, the intention is to cover all modifications, alternative constructions, and equivalents falling within the scope of the invention, as defined in the appended claims.

The use of the terms "a" and "an" and "the" and similar referents in the context of describing the invention (especially in the context of the following claims) are to be construed to cover both the singular and the plural, unless otherwise indicated herein or clearly contradicted by context. The terms "comprising," "having," "including," and "containing" are to be construed as open-ended terms (i.e., meaning "including, but not limited to,") unless otherwise noted. The term "connected" is to be construed as partly or wholly contained within, attached to, or joined together, even if there is something intervening. Recitation of ranges of values herein are merely intended to serve as a shorthand method of referring individually to each separate value falling within the range, unless otherwise indicated herein, and each separate value is incorporated into the specification as if it were individually recited herein. All methods described herein can be performed in any suitable order unless otherwise indicated herein or otherwise clearly contradicted by context. The use of any and all examples, or exemplary language (*e.g*., "such as") provided herein, is intended merely to better illuminate embodiments of the invention and does not pose a limitation on the scope of the invention unless otherwise claimed. No language in the specification should be construed as indicating any non-claimed element as essential to the practice of the invention.

Preferred embodiments of this invention are described herein, including the best mode known to the inventors for carrying out the invention. Variations of those preferred embodiments may become apparent to those of ordinary skill in the art upon reading the foregoing description.

## Claims

1. An embolic material filter catheter comprising:
an introducer sheath defining an introducer sheath lumen;
an inner sheath slidably disposed in the introducer sheath lumen and defining an inner sheath lumen;
an embolic material filter assembly having a proximal end portion and a distal end portion, wherein the proximal end portion is attached to a distal end portion of the inner sheath, wherein the embolic material filter assembly is reconfigurable between an insertion configuration and a deployed configuration, wherein the embolic material filter assembly is configured to interface with an inner surface of a blood vessel in the deployed configuration, and wherein the embolic material filter assembly is configured to filter embolic material from blood flowing through the embolic material filter assembly; and
a dilator assembly having a holding configuration and a non-holding configuration, wherein the dilator assembly comprises a dilator shaft, a dilator distal end member, and dilator pull wires; wherein the dilator shaft is configured to be extended through the inner sheath lumen, wherein the dilator distal end member is attached to a distal end of the dilator shaft, wherein a distal end portion of the embolic material filter assembly is restrained in the insertion configuration via engagement with the dilator pull wires and the dilator distal end member in the holding configuration, wherein the dilator pull wires are configured to be translated proximally relative to the dilator distal end member to reconfigure the dilator assembly from the holding configuration to the non-holding configuration to release the distal end portion of the embolic material filter assembly from engagement with the dilator pull wires and the dilator distal end member to reconfigure the embolic material filter assembly to the deployed configuration, and wherein the dilator assembly is removable from the inner sheath lumen while the embolic material filter assembly is in the deployed configuration via proximal retraction of the dilator assembly relative to the inner sheath.

2. The embolic material filter catheter of claim 1, wherein:
the dilator distal end member comprises dilator pull wire recesses;
each of the dilator pull wires comprises a respective dilator pull wire distal end portion that is disposed within a corresponding one of the dilator pull wire recesses when the dilator assembly is in the holding configuration; and
each of the dilator pull wire distal end portions is pulled out of the corresponding one of the dilator pull wire recesses during reconfiguration of the dilator assembly from the holding configuration to the non-holding configuration.

3. The embolic material filter catheter of claim 1, wherein the embolic material filter assembly is reconfigurable from the deployed configuration to a captured configuration in which the embolic material filter assembly is disposed in the introducer sheath lumen via proximal retraction of the inner sheath relative to the introducer sheath.

4. The embolic material filter catheter of claim 1, wherein the embolic material filter assembly can be restrained to conform to an outer surface of the dilator shaft from the proximal end portion of the embolic material filter assembly to the distal end portion of the embolic material filter assembly when the embolic material filter assembly is in the insertion configuration and disposed distal to the introducer sheath.

5. The embolic material filter catheter of claim 1, wherein:
the embolic material filter assembly has an outer surface that extends between the proximal end portion of the embolic material filter assembly and the distal end portion of the embolic material filter assembly; and
the outer surface of the embolic material filter assembly is disposable distal to the introducer sheath with the embolic material filter assembly in the insertion configuration.

6. The embolic material filter catheter of claim 1, wherein the embolic material filter assembly is configured to be retained in the insertion configuration at least partially via axial tension imparted into the embolic material filter assembly via the dilator assembly.

7. The embolic material filter catheter of claim 1, wherein:
the inner sheath accommodates insertion of a treatment catheter into the inner sheath lumen and advancement of a distal portion of the treatment catheter to a position distal to the distal end portion of the embolic material filter assembly in the deployed configuration; and
the distal end portion of the treatment catheter is configured for accomplishing a surgical task.

8. The embolic material filter catheter of claim 7, wherein:
the embolic material filter assembly is configured to, in the deployed configuration, interface with a patient's aorta and substantially block flow of embolic material through the patient's aorta past the embolic material filter assembly; and
the treatment catheter is configured for deploying a prosthetic aortic valve.

9. The embolic material filter catheter of claim 1, wherein the embolic material filter assembly comprises an outer scaffold and an inner filter mounted to the outer scaffold and configured to filter embolic material from blood flowing through the inner filter.

10. The embolic material filter catheter of claim 1, configured for coupling with an embolic material extraction device operable to draw embolic material through the inner sheath lumen while the embolic material filter assembly is in the deployed configuration.

11. The embolic material filter catheter of claim 1, wherein:
the embolic material filter assembly comprises an outer scaffold and an inner filter attached to the outer scaffold;
the outer scaffold is configured to radially expand into contact with a blood vessel along which embolic material is blocked from traversing; and
the inner filter is configured to prevent emboli of greater than a particular size from passing through the inner filter.

12. The embolic material filter catheter of claim 11, wherein the outer scaffold comprises distally extending loops of wires configured for atraumatic engagement of the blood vessel.

13. The embolic material filter catheter of claim 1, wherein:
a middle portion of the embolic material filter assembly has a middle portion external diameter in the deployed configuration;
the distal end portion of the embolic material filter assembly has a distal end portion external diameter in the deployed configuration; and
the middle portion external diameter is less than the distal end portion external diameter.

14. The embolic material filter catheter of claim 1, wherein:
the embolic material filter assembly comprises an outer scaffold and an inner filter; and
the inner filter is separated from the outer scaffold by an intervening annular space in the deployed configuration along a length of the inner filter.

15. The embolic material filter catheter of claim 1, wherein:
the embolic material filter assembly comprises an outer scaffold and an inner filter; and
a proximal end portion of the outer scaffold is formed by proximally extending unbraided wire segments of the outer scaffold that are bonded to an outer surface of the inner sheath,
wherein each of the proximally extending unbraided wire segments of the outer scaffold has a reduced diameter produced via electro-polishing.

## Patentansprüche

1. Filterkatheter für embolisches Material, umfassend:
eine Einführschleuse, die ein Lumen der Einführschleuse definiert;
einen Innenschaft, der verschiebbar in dem Lumen der Einführschleuse angeordnet ist und ein Lumen des Innenschafts definiert;
eine Filterbaugruppe für embolisches Material mit einem proximalen Endabschnitt und einem distalen Endabschnitt, wobei der proximale Endabschnitt an einem distalen Endabschnitt des Innenschafts befestigt ist, wobei die Filterbaugruppe für embolisches Material zwischen einer Einsetzkonfiguration und einer entfalteten Konfiguration rekonfiigurierbar ist, wobei die Filterbaugruppe für embolisches Material konfiguriert ist, um in der entfalteten Konfiguration mit einer Innenoberfläche eines Blutgefäßes eine Schnittstelle zu bilden, und wobei die Filterbaugruppe für embolisches Material konfiguriert ist, um embolisches Material aus dem Blut zu filtern, das durch die Filterbaugruppe für embolisches Material fließt; und
eine Dilatatorbaugruppe mit einer Haltekonfiguration und einer Nichthaltekonfiguration, wobei die Dilatatorbaugruppe einen Dilatatorschaft, ein distales Dilatatorendglied und Dilatatorzugdrähte umfasst; wobei der Dilatatorschaft konfiguriert ist, um durch das Lumen des Innenschafts verlängert zu werden, wobei das distale Dilatatorendglied an einem distalen Ende des Dilatatorschafts befestigt ist, wobei ein distaler Endabschnitt der Filterbaugruppe für embolisches Material in der Einsetzkonfiguration durch Eingriff mit den Dilatatorzugdrähten und dem distalen Dilatatorendglied in der Haltekonfiguration festgehalten wird, wobei die Dilatatorzugdrähte konfiguriert sind, um relativ zu dem distalen Dilatatorendglied proximal verschoben zu werden, um die Dilatatorbaugruppe von der Haltekonfiguration in die Nichthaltekonfiguration umzukonfigurieren, um den distalen Endabschnitt der Filterbaugruppe für embolisches Material aus dem Eingriff mit den Dilatatorzugdrähten und dem distalen Dilatatorendglied zu lösen, um die Filterbaugruppe für embolisches Material in die entfaltete Konfiguration umzukonfigurieren, und wobei die Dilatatorbaugruppe aus dem Lumen des Innenschafts entfernt werden kann, während sich die Filterbaugruppe für embolisches Material in der entfalteten Konfiguration befindet, indem die Dilatatorbaugruppe relativ zu dem Innenschaft proximal zurückgezogen wird.

2. Filterkatheter für embolisches Material nach Anspruch 1, wobei:
das distale Dilatatorendglied Aussparungen für die Dilatatorzugdrähte umfasst;
jeder der Dilatatorzugdrähte einen jeweiligen distalen Endabschnitt des Dilatatorzugdrahts umfasst, der in einer entsprechenden einen der Aussparungn für die Dilatatorzugdrähte angeordnet ist, wenn sich die Dilatatorbaugruppe in der Haltekonfiguration befindet; und
jeder der distalen Endabschnitte der Dilatatorzugdrähte während der Rekonfiiguration der Dilatatorbaugruppe von der Haltekonfiguration in die Nichthaltekonfiguration aus der entsprechenden einen der Aussparung für die Dilatatorzugdrähte herausgezogen wird.

3. Filterkatheter für embolisches Material nach Anspruch 1, wobei die Filterbaugruppe für embolisches Material von der entfalteten Konfiguration in eine eingefangene Konfiguration rekonfiigurierbar ist, in der die Filterbaugruppe für embolisches Material durch proximales Zurückziehen des Innenschafts relativ zu der Einführschleuse in dem Lumen der Einführschleuse angeordnet ist.

4. Filterkatheter für embolisches Material nach Anspruch 1, wobei die Filterbaugruppe für embolisches Material festgehalten werden kann, um sich von dem proximalen Endabschnitt der Filterbaugruppe für embolisches Material bis zu dem distalen Endabschnitt der Filterbaugruppe für embolisches Material an eine Außenoberfläche des Dilatatorschafts anzupassen, wenn sich die Filterbaugruppe für embolisches Material in der Einsetzkonfiguration befindet und distal zu der Einführhülse angeordnet ist.

5. Filterkatheter für embolisches Material nach Anspruch 1, wobei:
die Filterbaugruppe für embolisches Material eine Außenoberfläche aufweist, die sich zwischen dem proximalen Endabschnitt der Filterbaugruppe für embolisches Material und dem distalen Endabschnitt der Filterbaugruppe für embolisches Material erstreckt; und
die Außenoberfläche der Filterbaugruppe für embolisches Material distal zu der Einführschleuse angeordnet werden kann, wenn sich die Filterbaugruppe für embolisches Material in der Einsetzkonfiguration befindet.

6. Filterkatheter für embolisches Material nach Anspruch 1, wobei die Filterbaugruppe für embolisches Material konfiguriert ist, um wenigstens teilweise durch axiale Spannung, die über die Dilatatorbaugruppe auf die Filterbaugruppe für embolisches Material übertragen wird, in der Einsetzkonfiguration festgehalten zu werden.

7. Filterkatheter für embolisches Material nach Anspruch 1, wobei:
der Innenschaft das Einführen eines Behandlungskatheters in das Lumen des Innenschafts und das Vorschieben eines distalen Abschnitts des Behandlungskatheters in eine Position distal zu dem distalen Endabschnitt der Filterbaugruppe für embolisches Material in der entfalteten Konfiguration ermöglicht; und
der distale Endabschnitt des Behandlungskatheters konfiguriert ist, um eine chirurgische Aufgabe zu erfüllen.

8. Filterkatheter für embolisches Material nach Anspruch 7, wobei:
die Filterbaugruppe für embolisches Material konfiguriert ist, um in der entfalteten Konfiguration mit der Aorta eines Patienten eine Schnittstelle zu bilden und den Fluss von embolischem Material durch die Aorta des Patienten an der Filterbaugruppe für embolisches Material vorbei im Wesentlichen zu blockieren; und
der Behandlungskatheter konfiguriert ist, um eine Aortenklappenprothese zu entfalten.

9. Filterkatheter für embolisches Material nach Anspruch 1, wobei die Filterbaugruppe für embolisches Material ein äußeres Gerüst und ein inneres Filter umfasst, das an dem äußeren Gerüst montiert und konfiguriert ist, um embolisches Material aus dem durch das innere Filter fließende Blut zu filtern.

10. Filterkatheter für embolisches Material nach Anspruch 1, der für die Kopplung mit einer Vorrichtung für die Extraktion von embolischem Material konfiguriert ist, die betrieben werden kann, um embolisches Material durch das Lumen des Innenschafts zu saugen, während sich die Filterbaugruppe für embolisches Material in der entfalteten Konfiguration befindet.

11. Filterkatheter für embolisches Material nach Anspruch 1, wobei:
die Filterbaugruppe für embolisches Material ein äußeres Gerüst und ein inneres Filter umfasst, das an dem äußeren Gerüst befestigt ist;
das äußere Gerüst konfiguriert ist, um sich radial in einen Kontakt mit einem Blutgefäß auszudehnen, entlang dessen das embolische Material an dem Durchgang gehindert wird; und
das Innenfilter konfiguriert ist, um zu verhindern, dass Embolien, die größer als eine bestimmte Größe sind, das Innenfilter passieren.

12. Filterkatheter für embolisches Material nach Anspruch 11, wobei das äußere Gerüst sich distal erstreckende Drahtschlaufen umfasst, die für einen atraumatischen Eingriff in das Blutgefäß konfiguriert sind.

13. Filterkatheter für embolisches Material nach Anspruch 1, wobei:
ein Mittelabschnitt der Filterbaugruppe für embolisches Material in der entfalteten Konfiguration einen Außendurchmesser des Mittelabschnitts aufweist;
der distale Endabschnitt der Filterbaugruppe für embolisches Material in der entfalteten Konfiguration einen Außendurchmesser des distalen Endabschnitts aufweist; und
der Außendurchmesser des Mittelabschnitts kleiner ist als der Außendurchmesser des distalen Endabschnitts.

14. Filterkatheter für embolisches Material nach Anspruch 1, wobei:
die Filterbaugruppe für embolisches Material ein äußeres Gerüst und ein inneres Filter umfasst; und
das Innenfilter in der entfalteten Konfiguration entlang einer Länge des Innenfilters durch einen dazwischenliegenden Ringraum von dem Außengerüst getrennt ist.

15. Filterkatheter für embolisches Material nach Anspruch 1, wobei:
die Filterbaugruppe für embolisches Material ein äußeres Gerüst und ein inneres Filter umfasst; und
ein proximaler Endabschnitt des äußeren Gerüsts durch sich proximal erstreckende, nicht geflochtene Drahtsegmente des äußeren Gerüsts ausgebildet wird, die mit einer Außenoberfläche des Innenschafts verbunden sind, wobei jedes der sich proximal erstreckenden, nicht geflochtenen Drahtsegmente des äußeren Gerüsts einen durch Elektropolieren erzeugten reduzierten Durchmesser aufweist.

## Revendications

1. Cathéter de filtration d'emboles comprenant :
une gaine d'introduction définissant une lumière de gaine d'introduction ;
une gaine interne disposée de manière coulissante dans la lumière de gaine d'introduction et définissant une lumière de gaine interne ;
un ensemble de filtration d'emboles ayant une partie d'extrémité proximale et une partie d'extrémité distale, dans lequel la partie d'extrémité proximale est fixée à une partie d'extrémité distale de la gaine interne, dans lequel l'ensemble de filtration d'emboles est reconfigurable entre une configuration d'insertion et une configuration déployée, dans lequel l'ensemble de filtration d'emboles est configuré pour créer une interface avec une surface interne d'un vaisseau sanguin dans la configuration déployée, et dans lequel l'ensemble de filtration d'emboles est configuré pour filtrer des emboles issus du sang s'écoulant à travers l'ensemble de filtration d'emboles ; et
un ensemble de dilatateur ayant une configuration de maintien et une configuration de non-maintien, dans lequel l'ensemble de dilatateur comprend une tige de dilatateur, un élément d'extrémité distale de dilatateur et des fils de traction de dilatateur ; dans lequel la tige de dilatateur est configurée pour être étendue à travers la lumière de gaine interne, dans lequel l'élément d'extrémité distale de dilatateur est fixé à une extrémité distale de la tige de dilatateur, dans lequel une partie d'extrémité distale de l'ensemble de filtration d'emboles est maintenue dans la configuration d'insertion par mise en prise avec les fils de traction de dilatateur et l'élément d'extrémité distale de dilatateur dans la configuration de maintien, dans lequel les fils de traction de dilatateur sont configurés pour être translatés de manière proximale par rapport à l'élément d'extrémité distale de dilatateur pour reconfigurer l'ensemble de dilatateur de la configuration de maintien à la configuration de non-maintien afin de libérer la partie d'extrémité distale de l'ensemble de filtration d'emboles de la mise en prise avec les fils de traction de dilatateur et l'élément d'extrémité distale de dilatateur pour reconfigurer l'ensemble de filtration d'emboles selon la configuration déployée, et dans lequel l'ensemble de dilatateur est apte à être retiré de la lumière de gaine interne tandis que l'ensemble de filtration d'emboles est dans la configuration déployée par rétraction proximale de l'ensemble de dilatateur par rapport à la gaine interne.

2. Cathéter de filtration d'emboles selon la revendication 1, dans lequel :
l'élément d'extrémité distale de dilatateur comprend des évidements pour fils de traction de dilatateur ;
chacun des fils de traction de dilatateur comprend une partie d'extrémité distale de fil de traction de dilatateur respective qui est disposée à l'intérieur d'un évidement correspondant parmi les évidements pour fils de traction de dilatateur lorsque l'ensemble de dilatateur est dans la configuration de maintien ; et
chacune des parties d'extrémité distale de fil de traction de dilatateur est retirée de l'évidement correspondant parmi les évidements pour fils de traction de dilatateur pendant la reconfiguration de l'ensemble de dilatateur de la configuration de maintien à la configuration de non-maintien.

3. Cathéter de filtration d'emboles selon la revendication 1, dans lequel l'ensemble de filtration d'emboles est reconfigurable de la configuration déployée à une configuration de capture dans laquelle l'ensemble de filtration d'emboles est disposé dans la lumière de gaine d'introduction par rétraction proximale de la gaine interne par rapport à la gaine d'introduction.

4. Cathéter de filtration d'emboles selon la revendication 1, dans lequel l'ensemble de filtration d'emboles peut être maintenu de manière à suivre une surface externe de la tige de dilatateur de la partie d'extrémité proximale de l'ensemble de filtration d'emboles jusqu'à la partie d'extrémité distale de l'ensemble de filtration d'emboles lorsque l'ensemble de filtration d'emboles est dans la configuration d'insertion et disposé de manière distale par rapport à la gaine d'introduction.

5. Cathéter de filtration d'emboles selon la revendication 1, dans lequel :
l'ensemble de filtration d'emboles a une surface externe qui s'étend entre la partie d'extrémité proximale de l'ensemble de filtration d'emboles et la partie d'extrémité distale de l'ensemble de filtration d'emboles ; et
la surface externe de l'ensemble de filtration d'emboles est apte à être disposée de manière distale par rapport à la gaine d'introduction avec l'ensemble de filtration d'emboles dans la configuration d'insertion.

6. Cathéter de filtration d'emboles selon la revendication 1, dans lequel l'ensemble de filtration d'emboles est configuré pour être maintenu dans la configuration d'insertion au moins partiellement grâce à une tension axiale exercée sur ledit ensemble de filtration d'emboles par l'intermédiaire de l'ensemble de dilatateur.

7. Cathéter de filtration d'emboles selon la revendication 1, dans lequel :
la gaine interne accueille l'insertion d'un cathéter de traitement dans la lumière de gaine interne et l'avancement d'une partie distale du cathéter de traitement jusqu'à une position distale par rapport à la partie d'extrémité distale de l'ensemble de filtration d'emboles dans la configuration déployée ; et
la partie d'extrémité distale du cathéter de traitement est configurée pour accomplir une intervention chirurgicale.

8. Cathéter de filtration d'emboles selon la revendication 7, dans lequel :
l'ensemble de filtration d'emboles est configuré pour, dans la configuration déployée, créer une interface avec l'aorte d'un patient et bloquer sensiblement un flux d'emboles dans l'aorte du patient au-delà de l'ensemble de filtration d'emboles ; et
le cathéter de traitement est configuré pour déployer une valve aortique prothétique.

9. Cathéter de filtration d'emboles selon la revendication 1, dans lequel l'ensemble de filtration d'emboles comprend une structure externe et un filtre interne monté sur la structure externe et configuré pour filtrer des emboles issus du sang circulant à travers le filtre interne.

10. Cathéter de filtration d'emboles selon la revendication 1, configuré pour être couplé à un dispositif d'extraction d'emboles apte à être utilisé pour retirer des emboles à travers la lumière de gaine interne tandis que l'ensemble de filtration d'emboles est dans la configuration déployée.

11. Cathéter de filtration d'emboles selon la revendication 1, dans lequel :
l'ensemble de filtration d'emboles comprend une structure externe et un filtre interne fixé à la structure externe ;
la structure externe est configurée pour s'étendre radialement en contact avec un vaisseau sanguin le long duquel les emboles sont empêchés de passer ; et
le filtre interne est configuré pour empêcher des emboles de dimension supérieure à une taille donnée de passer à travers le filtre interne.

12. Cathéter de filtration d'emboles selon la revendication 11, dans lequel la structure externe comprend des boucles de fils s'étendant de manière distale, configurées pour une mise en prise atraumatique du vaisseau sanguin.

13. Cathéter de filtration d'emboles selon la revendication 1, dans lequel :
une partie centrale de l'ensemble de filtration d'emboles a un diamètre externe de partie centrale dans la configuration déployée ;
la partie d'extrémité distale de l'ensemble de filtration d'emboles a un diamètre externe de partie d'extrémité distale dans la configuration déployée ; et
le diamètre externe de partie centrale est inférieur au diamètre externe de partie d'extrémité distale.

14. Cathéter de filtration d'emboles selon la revendication 1, dans lequel :
l'ensemble de filtration d'emboles comprend une structure externe et un filtre interne ; et
le filtre interne est séparé de la structure externe par un espace annulaire intermédiaire dans la configuration déployée le long d'une longueur du filtre interne.

15. Cathéter de filtration d'emboles selon la revendication 1, dans lequel :
l'ensemble de filtration d'emboles comprend une structure externe et un filtre interne ; et
une partie d'extrémité proximale de la structure externe est formée par extension proximale de segments de fils non tressés de la structure externe qui sont liés à une surface externe de la gaine interne, dans laquelle chacun des segments de fils non tressés de la structure externe s'étendant de manière proximale a un diamètre réduit obtenu par électropolissage.
